(19) Europäisches Patentamt
European Patent Office
Office européen des brevets

(11) **EP 4 393 502 A2**

(12) **EUROPEAN PATENT APPLICATION**

(43) Date of publication:
**03.07.2024 Bulletin 2024/27**

(21) Application number: **24159081.9**

(22) Date of filing: **26.12.2019**

(51) International Patent Classification (IPC):
**A61K 38/00** (2006.01)

(52) Cooperative Patent Classification (CPC):
**A61P 25/14; A23L 33/18; A61K 8/64; A61Q 19/08; C07K 7/06;** A61K 38/00

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.12.2018 KR 20180169425**
**24.12.2019 KR 20190173644**

(62) Document number(s) of the earlier application(s) in accordance with Art. 76 EPC:
**19906010.4 / 3 904 368**

(71) Applicant: **Skinmed Co., Ltd.**
**Daejeon 34050 (KR)**

(72) Inventors:
• **KIM, Sung Hyun**
**Sejong-si 30098 (KR)**

• **CHOI, Won Il**
**Seoul 04500 (KR)**
• **SHIN, Yong Chul**
**Jinju-si 52817 (KR)**
• **LEE, Jeung Hoon**
**Daejeon 34124 (KR)**

(74) Representative: **Kraus & Lederer PartGmbB**
**Thomas-Wimmer-Ring 15**
**80539 München (DE)**

Remarks:
•The complete document including Reference Table(s) and the Sequence Listing(s) can be downloaded from the EPO website
•This application was filed on 22.02.2024 as a divisional application to the application mentioned under INID code 62.

(54) **ACETYLCHOLINE RECEPTOR INHIBITORY PEPTIDES AND USES THEREOF**

(57) The present invention relates to acetylcholine receptor inhibitory peptides and uses thereof, wherein phages with high binding affinity for acetylcholine receptors were screened using a random peptide recombinant phage, and acetylcholine receptor-binding peptides were selected through DNA of the phages. By having confirmed, using the selected peptides, the binding affinity for acetylcholine receptors and the effect of inhibiting the action of acetylcholine receptors, and having confirmed, via the modifications of the peptides, peptide sites and sequences that are vital in binding to acetylcholine receptors, the acetylcholine receptor inhibitory peptides of the present invention are expected to be used in the development of a cosmetic composition for alleviating wrinkles, a medicine for the prevention or treatment of acetylcholine receptor-associated diseases, and a health functional food for the amelioration thereof.

EP 4 393 502 A2

**Description**

Technical Field

The present invention relates to acetylene receptor inhibitory peptides and uses thereof.

Background Art

Acetylcholine, which is a chemical substance that is present in animal nervous tissues, is secreted at the nerve ending and serves to transmit the nerve stimulation to muscles. Transmitters secreted from nerve endings are known to be acetylcholine at the motor nerves and parasympathetic nerves and epinephrine (adrenaline) at the sympathetic nerves. The secretion of acetylcholine results in physiological actions, such as blood pressure lowering, heart rate suppression, intestinal contraction, and skeletal muscle contraction. For muscle contraction, the nerve sends a command to the muscle to contract, and thus the muscle contracts. In response to the command, the nerve secrets acetylcholine at the site where the nerve and the muscle meet each other (nerve-muscle junction), and this substance binds to the muscle's acetylcholine receptor, allowing the muscle to contract (Vincent, A., 1985; and Lindstrom, J.M., et al., 1976). The blockage of acetylcholine receptors at the peripheral site controlling femoral skeletal muscles causes muscular paralysis, and the blockage of acetylcholine receptors in smooth and cardiac muscles responsible for respiration or heat motion causes respiratory and cardiac paralysis.

Acetylcholine receptors are classified into muscarinic acetylcholine receptors (mAchR) and nicotinic acetylcholine receptors (nAchR). Muscarinic acetylcholine receptors are G protein-coupled receptors that can be activated by muscarine, and activate different signaling mechanisms depending on the subtype. Muscarinic acetylcholine receptors are distributed throughout the body, including the central nervous system and peripheral organs, and mainly serve to mediate physiological actions of acetylcholine secreted from postganglionic fibers of the parasympathetic nervous system.

Nicotinic acetylcholine receptors are receptors that mimic the pharmacological actions of nicotine and are ion channels operated by neurotransmitters. Nicotinic acetylcholine receptors are non-selective cation channels through which sodium, potassium, calcium ions, and the like pass non-selectively by opening and closing of ion channels, and regulate electronic signaling between nerve cells and muscle cells. Nicotinic acetylcholine receptors may be divided into a muscle type and a neuronal type according to the expression site. The muscle-type nicotinic acetylcholine receptors are expressed in the neuromuscular junction where motor neurons and skeletal muscles meet each other, and acetylcholine secreted from motor neurons contributes to induce an end plate potential (EPP) of Skeletal muscle cell membranes. Meanwhile, the neuronal nicotinic acetylcholine receptors are expressed in peripheral ganglia of the autonomic nervous system (ANS), and thus acetylcholine secreted from preganglionic fibers contributes to excite postganglionic fibers.

Drugs that hinder or inhibit the activity of acetylcholine or mimic the behavior of acetylcholine are very advantageously used. Acetylcholine receptor agonists are used to treat myasthenia gravis and Alzheimer's disease. Myasthenia gravis is an autoimmune disease in which antibodies to nicotinic acetylcholine receptors are produced in the body to inhibit normal acetylcholine signaling, and myasthenia gravis can be treated by increasing the time while acetylcholine can interact with each receptor, before inactivation, in the synaptic cleft between a nerve and a muscle, through acetylcholine esterase (AChE).

In addition, the obstruction of acetylcholine secretion suppresses muscle contraction to cause muscular paralysis and straighten wrinkles, for which Botox is used. Botox blocks the secretion of acetylcholine, which is a substance essential for muscle contraction at the motor nerve endings. As a result, the muscles are immobile, and the wrinkles induced by the muscles disappear. The muscle-relaxation effect by Botox gradually disappears after 3 to 6 weeks, and thus repeated administration is required.

Accordingly, the present inventors, while studying acetylcholine receptor-binding peptides, screened and secured peptides with high binding affinity to acetylcholine receptors, and verified that these peptides bind to acetylcholine reactors to prevent acetylcholine binding, thereby inhibiting the action of acetylcholine receptors, and thus, the present inventors could complete the present invention.

Furthermore, the present inventors identified predetermined arrangement orders of specific amino acids, which are important for inhibiting actions of acetylcholine receptors through the binding of the screened peptides and the acetylcholine receptors, and verified that libraries of the peptides set forth in predetermined formulas bind to acetylcholine receptors to inhibit the actions of the acetylcholine receptors, and thus the present inventors completed the present invention.

Korea Patent No. 1216008, which corresponds to a prior art, discloses a peptide that binds to an acetylcholine receptor and is selected using biopanning, but fails to disclose peptides and libraries containing the amino acid sequences of the present invention. In addition, Korean Patent Publication No. 2018-0028748 discloses a neurotransmitter release-controlling peptide containing acetylcholine and a wrinkle relief effect thereof, but fails to disclose the acetylcholine receptor binding affinity of the peptides containing amino acid sequences of the present invention and the resultant acetylcholine receptor inhibitory effect. Korean Patent Publication No. 2014-0139010 discloses a peptide for promoting percutaneous penetration, but has a different constitution from the inhibitory peptides through acetylcholine receptor binding of the present invention.

Detailed Description of the Invention

Technical Problem

An aspect of the present invention is to provide acetylcholine receptor inhibitory peptides and compositions containing the same.

Technical Solution

The present invention is directed to an acetylcholine receptor-binding peptide containing an amino acid sequence set forth in Formula 1 below:

[Formula 1]

$$X_L-(K \text{ or } R)-X-(K \text{ or } R)-X_M-(K \text{ or } R)-X_N$$

wherein $X_L$, $X_M$, and $X_N$ each may independently represent a sequence composed of one to eight arbitrary amino acids and X may represent a sequence composed of one arbitrary amino acid and, preferably, $X_L$, $X_M$, and $X_N$ each may independently represent one to four arbitrary amino acids and X may represent one arbitrary amino acid.

The present invention is directed to an acetylcholine receptor-binding peptide containing an amino acid sequence set forth in Formula 2 below:

[Formula 2]

$$(K \text{ or } R)-X-(K \text{ or } R)-(K \text{ or } R)-X_{M-1}-(K \text{ or } R)$$

wherein $X_{M-1}$ may represent a sequence composed of one to three arbitrary amino acids and X may represent a sequence composed of one arbitrary amino acid and, preferably, $X_{M-1}$ may represent three arbitrary amino acids and X may represent one arbitrary amino acid.

The peptide may be a peptide of any one sequence selected from the group consisting of SEQ ID NOs: 1 to 200 (see Table 10).

The present invention is directed to an acetylcholine receptor-binding peptide containing an amino acid sequence set forth in Formula 3 or 3-1 below:

[Formula 3]

$$X_L-(K \text{ or } R)-X-(K \text{ or } R)-(K \text{ or } R)-X_{M-1}-(K \text{ or } R)$$

wherein $X_L$ and $X_{M-1}$ each may independently represent a sequence composed of one to three arbitrary amino acids and X may represent a sequence composed of one arbitrary amino acid and, preferably, $X_L$ and $X_{M-1}$ each may independently represent three arbitrary amino acids and X may represent one arbitrary amino acid; and

[Formula 3-1]

$$(K \text{ or } R)-X-(K \text{ or } R)-(K \text{ or } R)-X_{M-1}-(K \text{ or } R)-X_N$$

wherein $X_{M-1}$ and $X_N$ each may independently represent a sequence composed of one to three arbitrary amino acids and X may represent a sequence composed of one arbitrary amino acid and, preferably, $X_{M-1}$ and $X_N$ each may independently represent three amino acids and X may represent one arbitrary amino acid.

The peptide may be a peptide of any one sequence selected from the group consisting of SEQ ID NOS: 201 to 400 (see Table 11).

The present invention is directed to an acetylcholine receptor-binding peptide containing an amino acid sequence set forth in Formula 4 below:

[Formula 4]

$$X_L-(K \text{ or } R)-X-(K \text{ or } R)-(K \text{ or } R)-X_{M-1}-(K \text{ or } R)-X_N$$

wherein $X_L$, $X_{M-1}$ and $X_N$ each may independently represent a sequence composed of one to three arbitrary amino acids and X may represent a sequence composed of one arbitrary amino acid and, preferably, $X_L$, $X_{M-1}$, and $X_N$ each may independently represent three arbitrary amino acids and X may represent one arbitrary amino acid.

The peptide may be a peptide of any one sequence selected from the group consisting of SEQ ID NOS: 401 to 600 (see Table 12).

The peptides may be composed of an 8- to 28-amino acid sequence. The number of amino acids is preferably 8 to 16, more preferably 8 to 14, still more preferably 11 to 16, and most preferably 14.

In the acetylcholine receptor-binding peptide, based on the amino acid sequence set forth in Formula 1, the 1st, 3rd, and 8th amino acids, which are K or R, may be important sites in acetylcholine receptor binding.

In the acetylcholine receptor-binding peptide, based on the amino acid sequence set forth in Formula 2, the 1st, 3rd, 4th, and 8th amino acids, which are K or R, may be important sites in acetylcholine receptor binding.

The amino acid sequences of the acetylcholine receptor-binding peptides exclude the amino acid sequences disclosed in Korean Patent No. 10-1971092, which corresponds to a prior patent of the present invention. The amino acid sequences disclosed in Korean Patent No. 10-1971092 include WTWKGRKSLLR.

The amino acid sequences of the acetylcholine receptor-binding peptides exclude the amino acid sequences disclosed in Korean Patent Publication No. 10-2014-0139010. The amino acid sequences disclosed in Korean Patent Publication No. 10-2014-0139010 are peptides of $(gly)_{n1}-(arg)_{n2}$, $(gly)_p-RGRDDRRQRRR-(gly)_q$, $(gly)_p-YGRKKRR-QRRR-(gly)_q$, and $(gly)_p-RKKRRQRRR-(gly)_q$, wherein the subscripts p and q each are independently an integer of 0 to 20; the subscript n1 is independently an integer of 1 to 8; and the subscript n2 is independently an odd number from 7 to 17.

The present invention is directed to acetylcholine receptor-binding peptides obtained by modifying the N-terminus or C-terminus of the above-described peptides.

The N-terminus or C-terminus may be modified by palmitoylation, acetylation, amidation, formylation or PEGylation, or by a linkage of at least one selected from the group consisting of 2-mercaptoacetic acid, 3-mercaptopropionic acid, 6-mercaptohexanoic acid, pyroglutamic acid, succinimide acid, cystramine, cysteamine, methyl ester, ethyl ester, benzyl ester, myristic acid, stearic acid, palmitic acid, cholesterol, 6-amino hexanoic acid, and 8-amino octanoic acid.

The present invention is directed to an acetylcholine receptor-binding peptide.

As used herein, the term "peptide" refers to a polymer consisting of two or more amino acids joined together by amide linkage or peptide linkage.

As used herein, the term "amino acid" includes all natural amino acids and other amino acids, for example, L- and D-isomers of natural amino acids, unnatural amino acids, amino acids not encoded by nucleotide sequences, and the like, which are used to prepare synthetic peptides in the field of peptides.

The natural amino acids may be alanine (Ala, A), cysteine (Cys, C), aspartic acid (Asp, D), glutamic acid (Glu, E), phenylalanine (Phe, F), glycine (Gly, G), histidine (His, H), isoleucine (Ile, I), lysine (Lys, K), leucine (Leu, L), methionine (Met, M), asparagine (Asn, N), proline (Pro, P), glutamine (Gln, Q), arginine (Arg, R), serine (Ser, S), threonine (Thr, T), valine (Val, V), tryptophan (Trp, W), and tyrosine (Tyr, Y).

The other amino acids may be 2-aminoadipic acid (2-aminohexanedioic acid), α-asparagine, 2-aminobutanoic acid, 2-aminocapric acid (2-aminodecanoic acid), α-glutamine, α-aminoisobutyric acid (α-methyl alanine), 2-aminopimelic acid (2-aminohepanedioic acid), γ-amino-β-hydroxybenzenepentanoic acid, 2-aminosuberic acid (2-aminooctanedioic acid), 2-carboxyazetidine, β-alanine, β-aspartic acid, 3,6-diaminohexanoic acid (β-lysine), butanoic acid, 4-amino-3-hydroxybutanoic acid, γ-amino-β-hydroxycyclohexanepentanoic acid, 3-cyclohexylalanine, N5-aminocarbonylornithine, 3-sulfoalanine, 2,3-diaminopropanoic acid, 2,7-diaminosuberic acid (2,7-diaminooctanedioic acid), S-ethylthiocysteine, γ-glutamic acid, γ-carboxylglutamic acid, hydroxyacetic acid (glycolic acid), pyroglutamic acid, homogrrginine, homocysteine, homohistidine, 2-hydroxyisovaleric acid, homoserine, 2-hydroxypentanoic acid, 5-hhydroxylysine, 4-hydroxyproline, isovaline, 2-hydroxypropanoic acid (lactic acid), mercaptoacetic acid, mercaptobutanoic acid, 3-hydroxy-4-methylproline, mercaptopropanoic acid, 3-naphthylalanine, norleucine, nortyrosine, norvaline, 2-carboxyoctahydroindole, ornithine, penicillamine (3-mercaptovaline), 2-phenylglycine, 2-carboxypiperidine, sarcosine (N-methylglycine), 1-amino-1-carboxycyclopentane, statin (4-amino-3-hydroxy-6-methylheptanoic acid), 3-thienylalanine, 3-carboxyisoquinoline, 3-methylvaline, ε-N-trimethyllysine, 3-thiazolylalanine, α-amino-2,4-dioxopyrimidinepropanoi c acid, and the like.

The peptides of the present invention may be prepared by the methods widely known in the art. Specifically, the peptides of the present invention may be prepared by using genetic recombination or protein expression systems, or may be prepared by a method of synthesis in vitro through chemical synthesis, such as peptide synthesis, and a cell-free protein synthesis method. More specifically, the peptides may be produced by well-known methods in the art, for example, an automated peptide synthesizer, and may be produced by genetic manipulation technology, but is not limited thereto. For example, a desired peptide may be produced by preparing a gene encoding a fusion protein composed of a fusion partner and the peptide of the present invention through genetic manipulation; transforming the prepared gene into a host microorganism; expressing the gene in the form of a fusion protein in the host microorganism; and cleaving and isolating the peptide of the present invention from the fusion protein via protease or compounds.

The peptides of the present invention may be present in the form of a salt. A salt form usable in the present invention may be prepared during the final isolation and purification of compounds or by reaction of an amino group with an appropriate acid. Examples of an acid addition salt may be acetates, adipates, alginates, citrates, aspartates, benzoates, benzenesulfonates, bisulfates, butyrates, camphorates, camphorsulfonates, digluconates, glycerophosphates, hemisulfates, heptanoates, hexanoates, formates, fumarates, hydrochloride, hydrobromide, hydroiodide, 2-hydroxyethanesulfonate, lactates, maleates, mesitylene sulfonate, methane sulfonate, naphthylene sulfonate, nicotinates, 2-naphthalenesulfonate, oxalates, pamoates, pectinates, persulfates, 3-phenylpropionate, picrates, pivalates, propionates, succinates, tartrates, trichloroacetates, trifluoroacetates, phosphates, glutamates, bicarbonates, para-toluene sulfonates, and undecanoates, but are not limited thereto. Also, examples of acids usable to form the acid addition salts may include inorganic acids, such as hydrochloric acid, hydrobromic acid, sulfuric acid, and phosphoric acid; and organic acids, such as oxalic acid, maleic acid, succinic acid, and citric acid, but are not limited thereto.

With respect to the peptides, a targeting sequence, a tag, a labeled residue, or an amino acid sequence designed for a purpose of increasing stability of the peptides may be added; an antibody or an antibody fragment thereof, human serum albumin (HSA), and the like for increasing targeting, efficacy increase, or stability may be bound; and the N-termini or C-termini of the peptides may be modified.

The term "antibody" refers to a specific protein molecule that is directed to an antigenic site. Preferably, the antibody refers to an antibody that specifically binds to a specific protein or an immunogenic fragment thereof, and may include all of monoclonal antibodies (mAb), polyclonal antibodies (pAb), and recombinant antibodies. The antibody may be easily produced using a known technique widely known in the art.

The antibody may include a complete form having two full-length light chains and two full-length heavy chains as well as a functional fragment of an antibody molecule. The functional fragment of the antibody molecule refers to a fragment retaining at least an antigen-binding function, and includes Fab, F(ab'), F(ab')$_2$, F(ab)$_2$, Fv, and the like.

The peptides may be encapsulated or immobilized in nanoparticles, microparticles, metal particles, ceramic particles, hydrogels, and the like, for delivery to specific tissues or to ensure stability, but are not limited thereto.

The nanoparticles, microparticles, metal particles, ceramic particles, hydrogels, and the like may be biocompatible and non-toxic.

As used herein, the term "acetylcholine receptor (AchR)" refers to a receptor to which acetylcholine secreted from the nerve endings is bound, and serves as a route for transmitting nerve stimulation by acetylcholine. For example, when in need of muscle contraction, the nerve sends a command to the muscle to contract, the nerve allows the secretion of acetylcholine at the site where the nerve and the muscle meet each other, and the secreted acetylcholine binds to the muscle's acetylcholine receptor, allowing the muscle to contract.

The acetylcholine receptors in the present invention are classified into muscarinic acetylcholine receptors and nicotinic acetylcholine receptors, and the acetylcholine receptors in the present invention are preferably nicotinic acetylcholine receptors.

The acetylcholine receptor-binding peptides bind to acetylcholine receptors, thereby preventing the binding of acetylcholine to the receptors, and thus can inhibit the actions of the acetylcholine receptors. Preferably, the peptides can relieve wrinkles and suppress abnormal muscle contraction by inhibiting muscle contraction; and, during surgery, can secure the convenience of surgery by promoting muscle relaxation.

Furthermore, the present invention provides a polynucleotide encoding the acetylcholine receptor-binding peptide. A polynucleotide containing a nucleotide sequence that is homologous to the nucleotide sequence constituting the polynucleotide may also be included in the scope of the polynucleotide provided in the present invention as long as the polynucleotide can encode a peptide capable of exhibiting binding activity to the acetylcholine receptor. Such a polynucleotide is preferably a polynucleotide containing an amino acid sequence showing at least 80% homology, more preferably a polynucleotide containing an amino acid sequence showing at least 90% homology, and most preferably a polynucleotide containing an amino acid sequence showing at least 95% homology.

Furthermore, the present invention provides a cosmetic composition, for wrinkle relief, containing the acetylcholine receptor peptide.

The acetylcholine receptor-binding peptide can relieve wrinkles by inhibiting the action of the acetylcholine receptor to prevent muscle contraction.

The cosmetic composition may further contain the acetylcholine receptor-binding peptide, and an adjuvant commonly used in the field of cosmetics, for example, a hydrophilic or lipophilic gelling agent, a hydrophilic or lipophilic activator, a preservative, an antioxidant, a solvent, a flavoring agent, a filler, a blocker, a pigment, a deodorant, or a dye.

The amount of the adjuvant is an amount that is commonly used in the art and, in any case, the adjuvant and the proportion thereof may be for selected so as not to adversely affect desirable properties of the cosmetic composition according to the present invention.

The cosmetic composition for wrinkle relief may be prepared by further containing an additive.

The additive may be a moisturizer, a functional raw material, a thickener, a softener, an emulsifier, a surfactant, a pH adjuster, and the like.

The moisturizer may include glycerin, propylene glycol, butylene glycol, hyaluronic acid, a ceramide component, and the like, but is not limited thereto.

The thickener may include a polymer, xanthan gum, and guar gum, but is not limited thereto.

The softener may include mineral oil, shea butter, or paraffin, but is not limited thereto.

The emulsifier may include dimethicone, beeswax, and the like.

The cosmetic composition for wrinkle relief may be used by mixing with a raw material having a wrinkle relief effect.

The raw material having a wrinkle relief effect may include vitamin A, a vitamin A derivative (retinyl palmitate, retinyl acetate, etc.), adenosine, and polyethoxylated retinamide, but is not limited thereto.

The cosmetic composition may be in the formulation of at least one selected from the group consisting of a lotion, a skin softener, a skin toner, an astringent, a cream, a foundation, an essence, a pack, a mask pack, a soap, a body cleanser, a cleansing foam, a body oil, and a body lotion, but is not limited thereto.

The cosmetic composition may be used every day, and may also be used even for an undetermined period, and preferably, the amount of use, the number of times of use, and the period of the cosmetic composition may be adjusted according to user's age, skin condition, or skin type.

Furthermore, the present invention provides a pharmaceutical composition for prevention or treatment of an acetylcholine receptor hyperactivity-associated disease, the pharmaceutical composition containing the acetylcholine receptor-binding peptide.

The acetylcholine receptor-binding peptide can bind to an acetylcholine receptor to inhibit the activation of the acetylcholine receptor, thereby preventing or treating the acetylcholine receptor hyperactivity-associated disease.

The acetylcholine receptor hyperactivity-associated disease refers to a disease in which the muscle contracts abnormally excessively, and examples thereof may be cervical dystonia, limb dystonia, truncal dystonia, blepharospasm, spasticity, hemifacial spasm, strabismus, nystagmus, tics, chronic pain, chronic migraine, neurogenic bladder, detrusor-sphincter dyssynergia, achalasia cardia, hyperhidrosis, sialorrhea, pediatric cerebral palsy, post-stroke muscle stiffness, back pain, enlarged prostate, urinary incontinence, vocal cord nodules and correction, hemorrhoids, dentition, and the like.

In addition, the pharmaceutical composition can be used to secure the convenience of surgery by promoting muscle relaxation during surgery, and can be used as a therapeutic agent or adjuvant for diseases caused by nicotine addiction, used for wrinkle removal, and used for square jaw or calf correction, but is not limited thereto.

The pharmaceutical composition may contain the acetylcholine receptor-binding peptide and a pharmaceutically acceptable excipient.

The pharmaceutical composition may be formulated in the forms of: an oral formulation, such as a powder, granules, a tablet, a capsule, a suspension, an emulsion, a syrup, or an aerosol; an externally applied preparation; a suppository; and a sterile injectable solution, according to usual methods, respectively. Examples of a carrier, an excipient, and a diluent that may be contained in the pharmaceutical composition may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. The pharmaceutical composition may be prepared by using a diluent or an excipient that is usually used, such as a filler, an extender, a binder, a humectant, a disintegrant, or a surfactant. Solid preparations for oral administration include a tablet, a pill, a powder, granules, a capsule, and the like. These solid preparations may be prepared by mixing the acetylcholine receptor-binding peptide with at least one excipient, for example, starch, calcium carbonate, sucrose or lactose, gelatin, or the like. Also, a lubricant, such as magnesium stearate or talc, may be used in addition to simple excipients. Liquid preparations for oral administration correspond to a suspension, a liquid for internal use, an emulsion, a syrup, and the like, and may contain simple diluents that are frequently used, such as water and liquid paraffin, as well as several excipients, such as a humectant, a sweetener, a flavoring agent, and a preservative. Preparations for parenteral administration include a sterile aqueous solution, a non-aqueous solvent, a suspension, an emulsion, a freeze-drying agent, and a suppository. Examples of the non-aqueous solvent and suspension may include propylene glycol, polyethylene glycol, vegetable oils such as olive oil, injectable esters such as ethyl oleate, and the like. A base material for the suppository may include Witepsol, Macrogol, Tween 61, cocoa butter, laurin butter, glycerogelatin, and the like.

Although not particularly limited to the formulation, the pharmaceutical composition may be used as an externally-applied preparation for skin, having one formulation selected from an ointment agent, a lotion agent, a spray agent, a patch agent, a cream agent, a gel agent, and a gel. The pharmaceutical composition may contain an agent for increasing transdermal absorption, such as, but not limited to, dimethyl sulfoxide, dimethylacetamide, dimethylformamide, a surfactant, an alcohol, acetone, propylene glycol, or polyethylene glycol. The frequency of application may vary significantly depending on the age, sex, and weight of a subject to be treated, a specific disease or pathological condition to be treated, the severity of a disease or pathological condition, the route of administration, and the judgment of a prescriber. The frequency of application may range from once a month up to 10 times a day, preferably from once a week up to 4 times a day, more preferably from three times a week up to three times a day, still more preferably one or two times a day.

The pharmaceutical composition of the present invention may be administered to mammals, such as a rat, livestock, and a human, through various routes. All modes of administration may be expected, and for example, administration may be conducted orally, rectally, or by intravenous, intramuscular, subcutaneous, transdermal, endometrial, or intracerebrovascular injection. Preferably, administration may be conducted by transdermal injection.

Furthermore, the present invention is directed to a health functional food composition, containing the acetylcholine receptor-binding peptide, for alleviating an acetylcholine receptor hyperactivity-associated disease.

The health functional food composition may contain the acetylcholine receptor-binding peptide and a food acceptable food supplement additive.

The health functional food composition of the present invention includes forms of a tablet, a capsule, a pill, a liquid preparation, and the like, and examples of foods to which the acetylcholine receptor-binding peptide of the present invention can be added include various kinds of foods, beverages, gums, teas, vitamin complexes, and health functional foods.

In accordance with still another aspect of the present invention, there is provided a composition for a medicinal device, the composition containing the acetylcholine receptor-binding peptide.

The composition for a medicinal device may be a filler, but is not limited thereto.

As used herein, the term "filler" refers to a substance that can supplement skin tissues, and has the purpose of filling through injection for restoration of the resilient face, improvement of facial contour, and relief of wrinkles.

The composition for a medicinal device can relieve wrinkles by suppressing muscle contraction and can exhibit a contour improving effect, through the acetylcholine receptor-binding peptide, and microparticles, nanoparticles, and hydrogels, on which the acetylcholine receptor-binding peptide is immobilized, can be injected to fill tissue.

Advantageous Effects

The present invention relates to acetylcholine receptor inhibitory peptides and uses thereof, wherein phages having high binding affinity to acetylcholine receptors were screened using random peptide recombinant phages and acetylcholine receptor-binding peptides were selected through phage DNA. By using the selected peptides, the acetylcholine receptor binding affinity and acetylcholine receptor inhibitory effects were verified, and through the modification of peptides, the sites and sequences of the peptides, which are crucial to acetylcholine receptor binding, were identified.

Furthermore, the identified amino acid sequences of peptides were expressed by general formulas, on the basis of the crucial peptide sites and sequences, and the libraries of the peptides were constructed, and it was verified that the constructed libraries were generally bound to acetylcholine receptors to inhibit the action of the acetylcholine receptors.

It is therefore expected that the acetylcholine receptor inhibitory peptides of the present invention can be used to develop a cosmetic composition for wrinkle relief, a medicinal product for preventing or treating an acetylcholine receptor-associated disease, and a health functional food for alleviating an acetylcholine receptor-associated disease.

Brief Description of the Drawings

FIG. 1 shows the results of analyzing the acetylcholine receptor binding affinity of screened and selected peptides (Spep-1 to Spep-11).

FIG. 2 shows the results of investigating the acetylcholine receptor inhibitory effect of the selected peptides in TE671 cells. (A) to (C) show the acetylcholine receptor inhibitory effect by acetylcholine according to the concentrations of Synake, Spep-1, Spep-2, Spep-4, and Spep-10 peptides; and (D) shows the acetylcholine receptor inhibitory effect by nicotine.

FIG. 3 shows the cytotoxicity results of Synake, Spep-1, and Spep-2 peptides on TE671 cells. All had no cytotoxicity even at a high concentration of 100 $\mu$M.

FIG. 4 shows the results of investigating acetylcholine receptor binding affinity according to concentration of Spep-2.

FIG. 5 shows the results of investigating sites of the sequence, which are crucial to acetylcholine receptor binding according to the peptide size, by using peptides with both the truncated termini in the amino acid sequence of Spep-2. (A) shows the investigation results using peptides with the truncated N-terminus (Spep-2-ND1 to -ND5) and (B) shows the investigation results using peptides with the truncated C-terminus (Spep-2-CD1 to -CD5).

FIG. 6 shows the results of comparing acetylcholine receptor binding affinity of (A) Spep-2-ND3 and (B) Spep-2-ND5. Compared with the binding affinity of Spep-2 (11-mer), the binding affinity of down to Spep-ND3 (8-mer) showed no significant difference, but the binding affinity of Spep-2-ND5 (6-mer) was reduced by 48 times.

FIG. 7 shows the results of investigating crucial sites in acetylcholine receptor binding by using alanine-scanning based on the amino acid sequences of Spep-1-ND3 (A) and Spep-2-ND3 (B).

FIG. 8 shows the results of comparing the acetylcholine receptor binding affinity between palmitoyl-conjugated Spep-2 and Spep-2. It can be verified that the conjugation of palmitoyl enhanced the binding affinity.

FIG. 9 verifies that palmitoyl-Spep-2 formed micelles. Pal-Spep-2 formed micelles and enhanced the binding affinity by the avidity effect.

FIG. 10 shows the results of comparing the AchR inhibitory effect of Palmitoyl-Spep-2 at different concentrations with those of Spep-2, Synake, and Synake, and bungarotoxin. As the binding affinity of Pal-Spep-2 was enhanced, the inhibitory ability of Pal-Spep-2 was also enhanced by about 10 times compared with Spep-2.

FIG. 11 shows the cytotoxicity results of palmitoyl-conjugated Spep-2 on TE671 cells. Palmitoyl-conjugated Spep-2 showed no cytotoxicity even at a high concentration of 10 $\mu$M.

FIG. 12 shows the results of investigating the AchR binding affinity of peptides in which K was substituted with R and R was substituted with K or N in the amino acid sequence of Spep-2-ND3 as a wild type, by the same method as the surface plasmon resonance analysis. The activity was maintained in the substitution with K or R, but the activity was significantly reduced in the substitution with N.

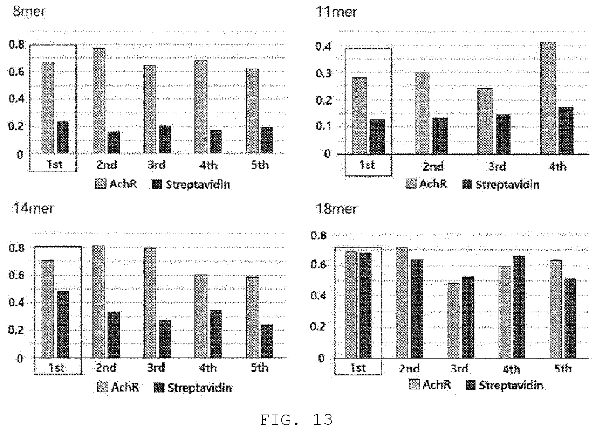

FIG. 13 shows the results of investigating binding affinity and specificity of phages to acetylcholine receptors, wherein the phages were screened in the biopanning of 8-mer L1 [((K or R)-X-(K or R) (K or R)-XXX-(K or R), X is a sequence composed of one arbitrary amino acid], 11-mer L2 [XXX (K or R)-X-(K or R) (K or R)-XXX-(K or R), X is a sequence of one arbitrary amino acid], 14-mer L3 [XXX (K or R)-X-(K or R) (K or R)-XXX- (K or R)XXX, X is a sequence of one arbitrary amino acid], and 18-mer L4[XXXXX(K or R)-X-(K or R) (K or R)-XXX-(K or R)XXXXX, X is a sequence of one arbitrary amino acid], which are peptide libraries optimized for acetylcholine receptors. The boxed parts are the first libraries, and it was verified that the first libraries among all of the L1 (8-mer), L2 (11-mer), and L3 (14-mer) libraries already showed high binding affinity and specificity to acetylcholine receptors, but the L4 (18-mer) library with a largest peptide size had no binding affinity and specificity to acetylcholine receptors.

FIG. 14 shows the result of analyzing the ratio of acetylcholine receptor absorbance to streptavidin absorbance when peptide phages specific to acetylcholine receptors were screened in the 4th and 5th biopanning of the optimized peptide L1 (8-mer) libraries. Spep-2 was also used as a control, and it was verified that all the peptides had higher binding affinity and specificity than Spep-2.

FIG. 15 shows the result of analyzing the ratio of acetylcholine receptor absorbance to streptavidin absorbance when peptide phages specific to acetylcholine receptors were screened in the 4th and 5th biopanning of the optimized peptide L2 (11-mer) libraries. Spep-2 was also used as a control, and it was verified that all the peptides had higher binding affinity and specificity than Spep-2.

FIG. 16 shows the result of analyzing the ratio of acetylcholine receptor absorbance to streptavidin absorbance when peptide phages specific to acetylcholine receptors were screened in the 4th and 5th biopanning of the optimized peptide L3 (14-mer) libraries. Spep-2 was also used as a control, and it was verified that all the peptides had higher binding affinity and specificity than Spep-2.

FIG. 17 shows the results of comparing the binding affinity to acetylcholine receptors among 40 peptides with excellent specificity selected from the optimized peptide L1 (8-mer) library. All the optimized peptides showed higher binding affinity than Spep-2.

FIG. 18 shows the results of comparing the binding affinity to acetylcholine receptors among 40 peptides with excellent specificity selected from the optimized peptide L2 (11-mer) library. All the optimized peptides showed higher binding affinity than Spep-2.

FIG. 19 shows the results of comparing the binding affinity to acetylcholine receptors among 40 peptides with excellent specificity selected from the optimized peptide L3 (14-mer) library. All the optimized peptides showed higher binding affinity than Spep-2.

FIG. 20 shows the results of comparing the acetylcholine receptor inhibitory effect of 20 peptides in the highest order among 40 optimized L1 (8-mer) peptides. All the optimized peptides showed higher inhibitory ability than Spep-2.

FIG. 21 shows the results of comparing the acetylcholine receptor inhibitory effect of 20 peptides in the highest order among 40 optimized L2 (11-mer) peptides. All the optimized peptides showed higher inhibitory ability than Spep-2.

FIG. 22 shows the results of comparing the acetylcholine receptor inhibitory effect of 20 peptides in the highest order among 40 optimized L3 (14-mer) peptides. All the optimized peptides showed higher inhibitory ability than Spep-2.

FIG. 23 shows the results of comparing the acetylcholine receptor binding affinity among representative peptides with the highest affinity among the optimized peptides L1 (8-mer), L2 (11-mer), and L3 (14-mer).

FIG. 24 shows the results of comparing the acetylcholine receptor inhibitory between Spep-2 and the selected representative peptides according to the concentration.

FIG. 25 shows the results of comparing the multiples of acetylcholine receptor inhibitory effects between Spep-2 and the selected representative peptides. L3-37 peptide had an inhibitory ability enhanced by 32 times compared with Spep-2.

FIG. 26 shows the result of comparing the binding affinity to acetylcholine receptors through various modifications of a terminus of the representative L3-37 peptide.

FIG. 27 shows the result of comparing the binding affinity to acetylcholine receptors through various modifications of a terminus of the representative L3-28 peptide.

FIG. 28 shows the result of comparing the binding affinity to acetylcholine receptors through various modifications of a terminus of the representative L3-27 peptide.

FIG. 29 shows the result of comparing the binding affinity to acetylcholine receptors through various modifications of a terminus of the representative L2-110 peptide.

FIG. 30 shows the result of forming micelles by Myristic-L3-37 과 Stearic-L3-37.

FIG. 31 shows the results of comparing the AchR inhibitory effect of Palmitoyl-L3-37, Palmitoyl-L3-28, and Palmitoyl-L3-27, Palmitoyl-L2-110 at different concentrations compared with bungarotoxin.

FIG. 32 shows the cytotoxicity results of Palmitoyl-L3-37 on TE671 cells.

FIG. 33 shows the cytotoxicity results of Palmitoyl-L3-28 on TE671 cells.

FIG. 34 shows the cytotoxicity results of Palmitoyl-L3-27 on TE671 cells.

FIG. 35 shows the cytotoxicity results of Palmitoyl-L2-110 on TE671 cells.

FIG. 36 shows the results of animal efficacy assay for Palmitoyl-L3-37 peptide by using Catwalk equipment.

FIG. 37 shows the results of using DAS assay to perform animal efficacy assay of Palmitoyl-L3-37 peptide at different concentrations.

FIG. 38 shows the results of restoration of animal efficacy of Palmitoyl-L3-37 peptide after DAS assay.

FIG. 39 shows the results of animal acute toxicity assay of Palmitoyl-L3-37 peptide.

Mode for Carrying Out the Invention

Hereinafter, preferable exemplary embodiments of the present invention will be described in detail. However, the present invention is not limited to the exemplary embodiments described herein and can be embodied in many different forms. Rather, these exemplary embodiments are provided so that the present disclosure will be thorough and complete and will fully convey the scope of the disclosure to those skilled in the art.

<Example 1: Acetylcholine receptor-binding peptide screening through biopanning>

The present inventors produced random recombinant phages through random peptide library DNA preparation and transformation and screened acetylcholine receptor (AchR)-binding peptides through biopanning, according to the methods described in a prior patent (Korean Patent No. 10-1971092). Table 1 shows the sequencing results of AchR-specific peptides selected from the input phages having high affinity and specificity to AchR in the 4th and 5th rounds of biopanning.

TABLE 1

| Peptide Name | Amino acid sequence |
|---|---|
| Spep-1 | WTWKGKGTLNR |
| Spep-2 | WTWKGRKSLLR |
| Spep-3 | WTWKGEDKGKN |
| Spep-4 | WTWKGRDKLQM |
| Spep-5 | WTWKGQLGQLS |
| Spep-6 | WTWKGGRLSAS |
| Spep-7 | WTWKGRQLNNQ |
| Spep-8 | WTWKGDNLQNN |
| Spep-9 | WTWKGLYQRLG |

(continued)

| Peptide Name | Amino acid sequence |
| --- | --- |
| Spep-10 | WTWKGNKQVKF |
| Spep-11 | WTWKGETYDSK |

<Example 2: Investigating acetylcholine receptor binding affinity of peptides>

Spep-1 to Spep-11 selected in Example 1 were synthesized, and the AchR binding affinity was compared thereamong using a surface plasmon resonance (SPR) analyzer (Biacore3000, Biacore A, Sweden).

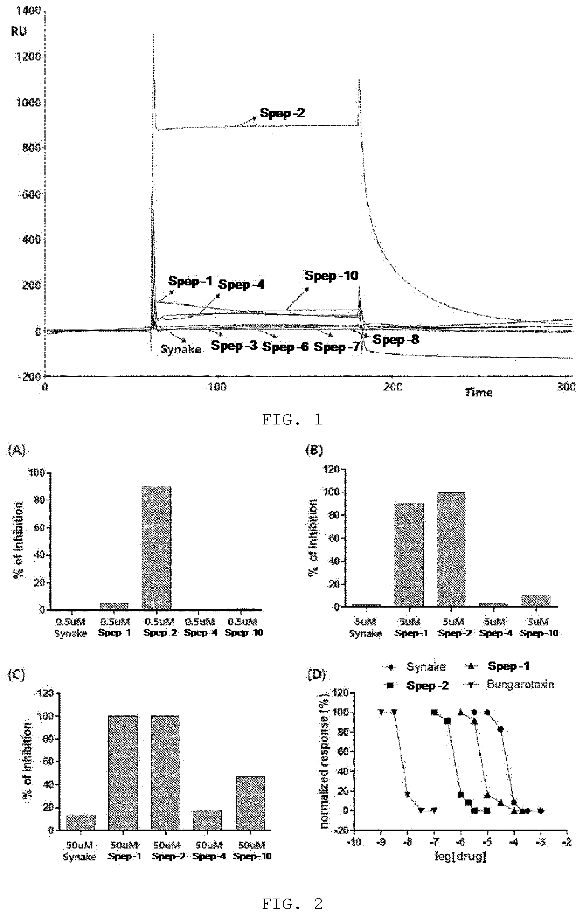

Acetylcholine receptor proteins were immobilized on CM5 chips, which are biosensor chips for surface plasmon resonance analysis, using EDC/NHS, and then analysis was performed under conditions of 20 mM Tris (pH 7.0) running buffer, rate of 30 $\mu$l/min, and 10 $\mu$M peptides (Spep-1 to Spep-11). The association and dissociation were observed for up to 500 seconds, and the results are shown in FIG. 1. Synake known to relieve wrinkles by binding to AchR was used as a positive control.

As shown in FIG. 1, Spep-1, Spep-2, Spep-4, and Spep-10 showed high AchR binding affinity compared with Synake known to bind to AchR.

<Example 3: Investigating acetylcholine receptor inhibition of peptides>

To investigate AchR inhibitory effects of the peptides having high acetylcholine receptor binding affinity in Example 2, AchR-overexpressed TE671 cells were used.

TE671 cells were cultured for 4 days using DMEM containing 10% fetal bovine serum (FBS) and 1% penicillin/streptomycin. The cultured cells were detached by trypsinization, and then inoculated at 2 $\times 10^4$ cells/well in 12-well cell culture plates with 18-mm cover slides placed therein, and cultured for 4 days. The cover slides with the cultured cells were transferred in new 12-well plates, and 997 $\mu$l of Hanks' balanced salt solution and 3 $\mu$l of Fura-2-AM (intracellular calcium ion indicator) were added thereto, followed by culture in a 5% CO2 incubator at 37°C for 15 minutes. Thereafter, the cells were washed three or four times with HBSS buffer to remove the remaining Fura-2-AM, and then 1 ml of HBSS buffer was added thereto. The cover slides with cells grown thereon were inserted into a microscopic observation chamber, and then 500 ul of HBSS buffer was added thereto. Thereafter, the peptides selected in Example 2 were added at 0.5, 5, or 50 $\mu$M, and then left for about 3 minutes so as to bind to AchR. Thereafter, the fluorescence intensity of Fura-2 in the cells was measured by addition of 125 $\mu$M nicotine or 1 $\mu$M acetylcholine, to thereby investigate the action of AchR. Synake and bungarotoxin were used as positive controls. The results are shown in FIG. 2.

As shown in FIG. 2, as for acetylcholine treatment (A to C), Spep-1 showed an AchR inhibitory effect of 5% at 0.5 $\mu$M, 90% at 5 $\mu$M, and 100% at 100 $\mu$M; and Spep-2 showed an AchR inhibitory effect of 90% at 0.5 $\mu$M and 100% at 5 $\mu$M; and Spep-4 and Spep-10 showed AchR inhibitory effects of 17% and 47% at 50 $\mu$M, respectively. As for nicotine treatment (D), Spep-1 and Spep-2, compared with Synake, showed excellent nicotinic acetylcholine receptor inhibitory effects.

It can be therefore seen that Spep-1 and Spep-2 had excellent AchR inhibitory effects, and especially, Spep-2 had the highest binding affinity to AchR, leading to the highest AchR inhibitory effect.

<Example 4: Measuring cytotoxicity of Synake, Spep-1, and Spep-2>

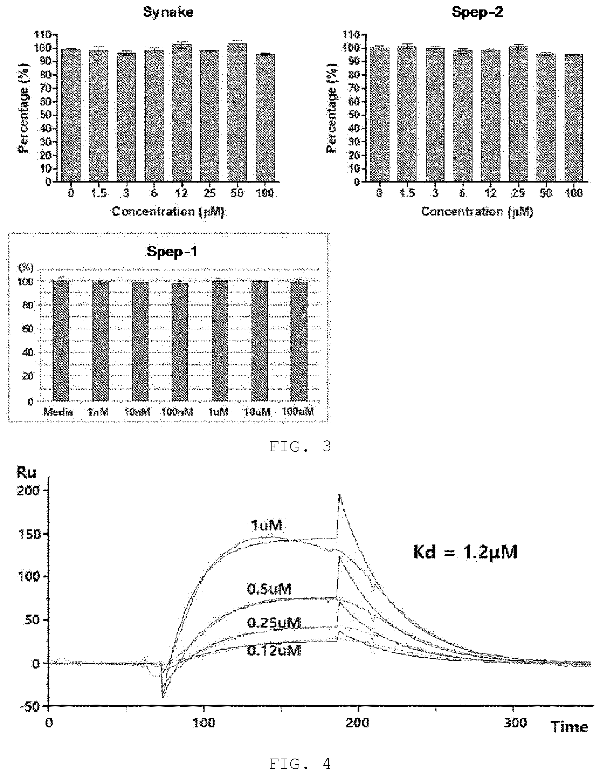

To evaluate the toxicity of Spep-1 and Spep-2, WST Assay was performed on TE671 cells. The toxicity of Synake was also evaluated, and the results are shown in FIG. 3.

As shown in FIG. 3, Spep-1, Spep-2, and Synake showed no toxicity even at a treatment concentration of 100 $\mu$M.

<Example 5: Investigating acetylcholine receptor binding affinity of Spep-2 peptide with highest binding affinity>

To investigate the AchR binding affinity of Spep-2 with highest AchR binding affinity in Example 2, the dissociation constant (Kd) was analyzed using the surface plasmon analyzer in Example 2 by the same method, and the results are shown in FIG. 4. The treatment concentrations of Spep-2 were 0.12 to 1 $\mu$M, and the dissociation constant was compared while Synake was used as a positive control.

As shown in FIG. 4, the association curves rapidly increased as Spep-2 was allowed to flow, and the association curves increased in a dependent manner of the treatment concentration of Spep-2. After Spep-2 was allowed to completely flow, only a running buffer was allowed to flow for dissociation, during which the increased association curves were not reduced, indicating that Spep-2 was continuously bound to AchR.

The Kd value for AchR of Spep-2 was analyzed to be 1.2 $\mu$M, indicating that the AchR binding affinity was about 2,000 times superior to that of Synake, considering that the kd value of Synake was 2,300 $\mu$M.

<Example 6: Investigating crucial site of peptide sequence in acetylcholine receptor binding>

Example 6-1: Investigating acetylcholine receptor binding affinity through sequential deletion of amino acids from terminus

To identify the sites of a peptide, which play an important role in AchR binding, peptide modification was conducted based on the amino acid sequence of Spep-2 identified in Example 1 and the modified peptide was applied to the surface plasmon resonance analyzer in Example 2 to investigate the binding affinity to AchR.

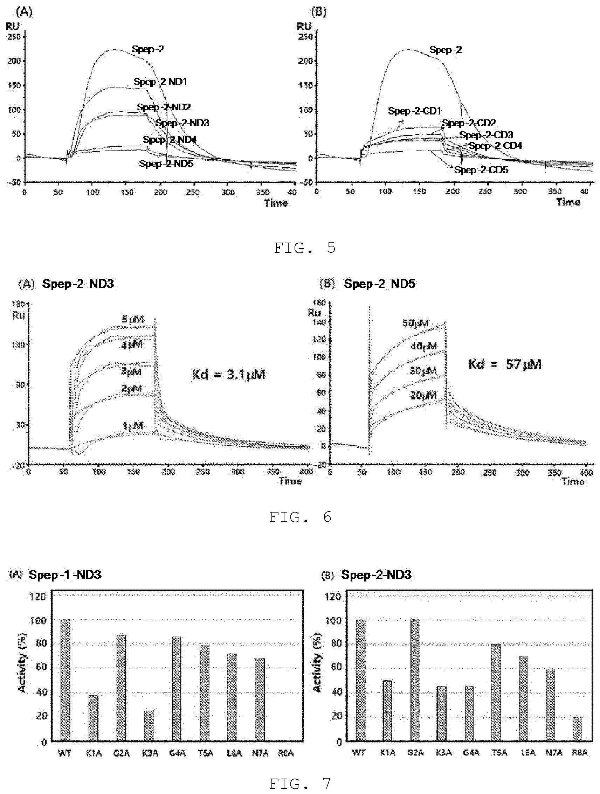

Based on the amino acid sequence of Spep-2, amino acids from the N-terminus or C-terminus of the peptide were stepwise deleted to synthesize the peptides shown in Table 2 below, and the AchR binding affinity of the synthesized peptides were compared, and the results are shown in FIG. 5.

TABLE 2

| Peptide name | Amino acid sequence |
|---|---|
| Spep-2 | WTWKGRKSLLR |
| Spep-2-ND1 | TWKGRKSLLR |
| Spep-2-ND2 | WKGRKSLLR |
| Spep-2-ND3 | KGRKSLLR |
| Spep-2-ND4 | GRKSLLR |
| Spep-2-ND5 | RKSLLR |
| Spep-2-CD1 | WTWKGRKSLL |
| Spep-2-CD2 | WTWKGRKSL |
| Spep-2-CD3 | WTWKGRKS |
| Spep-2-CD4 | WTWKGRK |
| Spep-2-CD5 | WTWKGR |

As shown in FIG. 5, in cases where the amino acids from the N-terminus of Spep-2 were deleted stepwise (A), Spep-2-ND1 to -ND3 showed a reduction in AchR binding affinity by about 50% compared with Spep-2, but still maintained the binding affinity to AchR, whereas Spep-2-ND4 and -ND5 showed significantly reduced binding affinity. In cases where the amino acids from the C-terminus of Spep-2 were deleted stepwise (B), all of Spep-2-CD1 to -CD5 showed rapid reductions in AchR binding.

It can be therefore seen that the 4th amino acid K (Lys) and 11th amino acid R (Arg) in the amino acid sequence of Spep-2 are crucial sites in AchR binding.

Example 6-2: Investigating acetylcholine receptor binding affinity of modified peptides

Among the modified peptides of Spep-2 identified in Example 6-1, Spep-2-ND3, which was identified to have the minimum sequence necessary for binding to AchR, and Spep-2-ND5, which had a smaller size than Spep-2-ND3, were applied to the surface plasmon resonance analyzer in Example 2 to investigate the binding affinity to AchR, and the results are shown in FIG. 6.

As shown in FIG. 6, Spep-2-ND3 (A) showed higher binding affinity to AchR even at low concentrations compared with Spep-2-ND5 (B), and as a result of investigating the dissociation constant, Spep-2-ND3 was 3.1 $\mu$M, whereas Spep-2-ND5 was 57 $\mu$M, indicating that Spep-2-ND3 had significantly excellent AchR binding affinity compared with Spep-2-ND5.

It can be therefore seen that the 11-mer Spep-2 and the 8-mer Spep-2-ND3 did not have a large difference in AchR binding affinity, but the 6-mer Spep-2-ND5 showed a remarkable reduction in AchR binding affinity. These results indicate that three amino acids from the N-terminus of Spep-2 were not significantly crucial to AchR binding affinity.

<Example 7: Identifying crucial sites of peptides in acetylcholine receptor binding by using alanine-scanning>

By using Spep-2-ND3 among the peptides identified in Example 6, and Spep-1-ND3 (KGKGTLNR), which has three amino acid deletions from the N-terminus of Spep-1 having high amino acid sequence similarity to Spep-2, alanine-scanning was conducted to identify crucial sites of each of the peptides.

Spep-1-ND3 and Spep-2-ND3 were set as a wild type, and the peptides in Table 3, in which alanine (Ala) substitution was sequentially conducted in the amino acid sequence of each peptide, were synthesized and then compared for AchR binding affinity by the same method as the surface plasmon resonance analysis in Example 2. The treatment concentration of each peptide was 20 $\mu$M, and the results are shown in FIG. 7.

TABLE 3

| Peptide name | Amino acid sequence | Peptide name | Amino acid sequence |
|---|---|---|---|
| Spep-1-ND3(WT) | KGKGTLNR | Spep-2-ND3(WT) | KGRKSLLR |
| Spep-1-ND3(K1A) | AGKGTLNR | Spep-2-ND3(K1A) | AGRKSLLR |
| Spep-1-ND3 (G2A) | KAKGTLNR | Spep-2-ND3 (G2A) | KARKSLLR |
| Spep-1-ND3 (K3A) | KGAGTLNR | Spep-2-ND3 (R3A) | KGAKSLLR |
| Spep-1-ND3 (G4A) | KGKATLNR | Spep-2-ND3 (K4A) | KGRASLLR |
| Spep-1-ND3(T5A) | KGKGALNR | Spep-2-ND3 (S5A) | KGRKALLR |
| Spep-1-ND3 (L6A) | KGKGTANR | Spep-2-ND3 (L6A) | KGRKSALR |
| Spep-1-ND3 (N7A) | KGKGTLAR | Spep-2-ND3(L7A) | KGRKSLAR |
| Spep-1-ND3 (R8A) | KGKGTLNA | Spep-2-ND3 (R8A) | KGRKSLLA |

As shown in FIG. 7, the peptides in which the K at the 1st site, K at the 3rd site, and R at the 8th site were substituted with A in Spep-1-ND3 showed a reduction in AchR binding affinity (A); and the peptides in which the K at the 1st site, R at the 3rd site, K at the 4th site, and R at the 8th site were substituted with A in Spep-2-ND3 showed a reduction in AchR binding affinity. It can be therefore seen that K and R sites in Spep-1-ND3 and Spep-2-ND3 are crucial sites in AchR binding.

<Example 8: Identifying acetylcholine receptor binding affinity of fatty acid derivative-conjugated Spep-2 according to peptide modification>

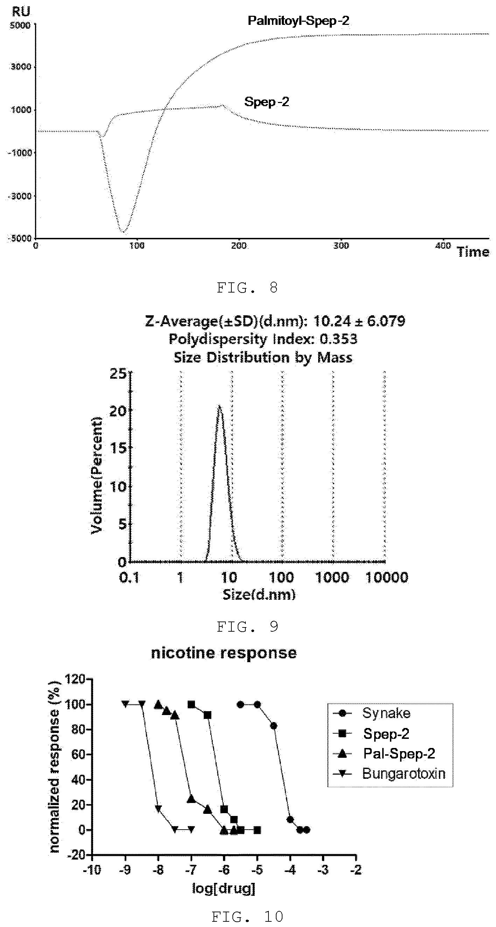

The change in AchR binding affinity according to the modification of peptides binding to AchR was investigated. Specifically, by using a peptide (Palmitoyl-Spep-2) obtained by conjugating palmitoyl as a fatty acid derivative to Spep-2 having the highest binding affinity to AchR among the peptides identified in Example 2, the AchR binding affinity was investigated by the same method as the surface plasmon resonance analysis in Example 2, and the results are shown in FIG. 8. The binding affinity between Spep-2 and AchR was used as a control.

As shown in FIG. 8, the AchR binding affinity of Palmitoyl-Spep-2 was stronger than that of Spep-2. The reason is that, as shown in FIG. 9, micelle structures in which the fat-soluble palmitoyl gather inward and the water-soluble Spep-2 is exposed outward bind to several sites of AchR to have an avidity effect.

<Example 9: Investigating acetylcholine receptor inhibition of Palmitoyl-Spep-2>

The AchR inhibitory effect of Palmitoyl-Spep-2, which has been verified to have excellent AchR binding affinity in Example 8, was investigated. The AchR inhibitory effect was investigated by the same method as in Example 3, and the treatment with Palmitoyl-Spep-2 was conducted according to the concentration. For comparison of the AchR inhibitory effect, the treatment with Spep-2, Synake, and bungarotoxin as controls was conducted according to the concentration, and the results are shown in FIG. 10.

As shown in FIG. 10, Palmitoyl-Spep-2 showed a higher inhibitory effect on AchR than Spep-2. More specifically, as for IC50 value for AchR in each peptide, Synake was 75 $\mu$M; Spep-2 was 750 nM; Palmitoyl-Spep-2 was 75 nM; and bungarotoxin was 7.5 nM, indicating that the AchR inhibitory effect of Pal-Spep-2 was 10 times excellent compared with that of Spep-2.

<Example 10: Measuring cytotoxicity of Palmitoyl-Spep-2>

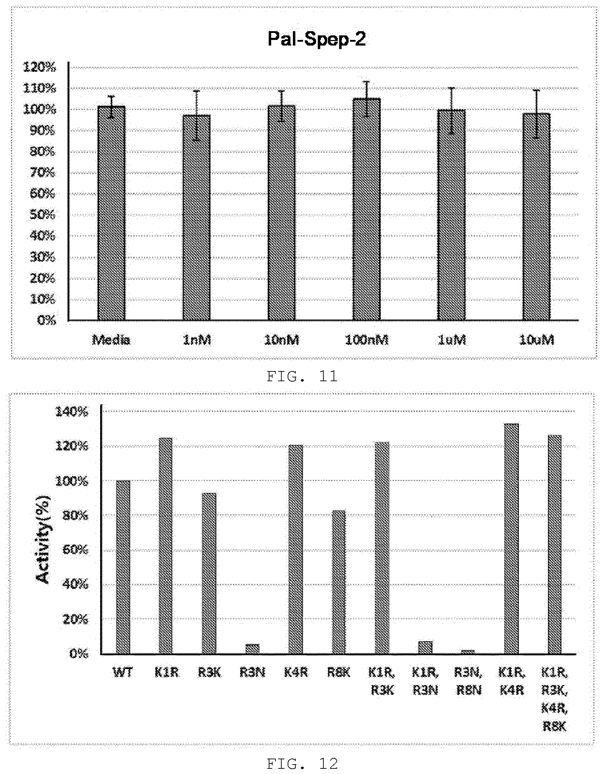

To evaluate cytotoxicity of Palmitoyl-Spep-2, WST assay was performed on TE671 cells, and the results are shown in FIG. 11.

As shown in FIG. 11, Palmitoyl-Spep-2 showed no cytotoxicity even at 10 $\mu$M.

<Example 11: Investigating change in AchR binding affinity through amino acid substitutions of K and R in amino acid sequence of Spep-2-ND3>

It was investigated whether the AchR binding affinity was changed when in the amino acids K and R of Spep-2-ND3, which have been identified to be crucial sites in AchR binding in Example 7, K was substituted R and R was substituted with K or N.

Spep-2-ND3 was set to a wild type, and the peptides in Table 4, in which K was substituted with R and R was substituted with K or N in the amino acid sequence of Spep-2-ND3, were synthesized and then compared for AchR binding affinity by the same method as the surface plasmon resonance analysis in Example 2. The treatment concentration of each peptide was 20 $\mu$M, and the results are shown in FIG. 12.

TABLE 4

| Peptide name | Amino acid sequence |
|---|---|
| Spep-2-ND3(WT) | KGRKSLLR |
| Spep-2-K1R | RGRKSLLR |
| Spep-2-R3K | KGKKSLLR |
| Spep-2-R3N | KGNKSLLR |
| Spep-2-K4R | KGRRSLLR |
| Spep-2-R8K | KGRKSLLK |
| Spep-2-K1R-R3K | RGKKSLLR |
| Spep-2-K1R-R3N | RGNKSLLR |
| Spep-2-R3N-R8N | KGNKSLLN |
| Spep-2-K1R-K4R | RGRRSLLR |
| Spep-2-K1R-R3K-K4R-R8K | RGKRSLLK |

As shown in FIG. 12, the peptides in which K was substituted with R in Spep-2-ND3 showed an increase in AchR binding affinity, and the peptides in which R was substituted with K showed a slight reduction in AchR binding affinity, but no significant difference. However, the peptides in which R was substituted with N showed a significant reduction in AchR binding affinity. It can be therefore seen that the substitution of K and R with each other in Spep-2-ND3 made no difference in AchR binding, but the substitution of R with N resulted in no AchR binding affinity.

<Example 12: Constructing peptide libraries for selection of optimized peptides with enhanced acetylcholine receptor binding affinity>

Example 12-1: Random peptide library DNA construction and transformation

Through the results identified in Examples 6, 7, and 11, the Spep-2-ND3 peptide sequence was modified to construct random peptide libraries of 8-mer; (K/R)X(K/R)(K/R)XXX(K/R), 9-mer; X(K/R)X(K/R)(K/R)XXX(K/R), (K/R)X(K/R)(K/R)XXX(K/R)X, 10-mer; XX(K/R)X(K/R)(K/R)XXX(K/R), (K/R)X(K/R)(K/R)XXX(K/R)XX, X(K/R)X(K/R)(K/R)XXX(K/R)X, 11-mer; XXX(K/R)X(K/R)(K/R)XXX(K/R), (K/R)X(K/R)(K/R)XXX(K/R)XXX, X(K/R)X(K/R)(K/R)XXX(K/R)XX, XX(K/R)X(K/R)(K/R)XXX(K/R)X, 12-mer; X(K/R)X(K/R)(K/R)XXX(K/R)XXX, XX(K/R)X(K/R)(K/R)XXX(K/R)XX, XXX(K/R)X(K/R)(K/R)XXX(K/R)X, 13-mer; XX(K/R)X(K/R)(K/R)XXX(K/R)XXX, XXX(K/R)X(K/R)(K/R)XXX(K/R)XX, 14-mer; XXX(K/R)X(K/R)(K/R)XXX(K/R)XXX, and 18-mer; XXXXX(K/R)X(K/R)(K/R)XXX(K/R)XXXXX ((K/R) is one of either K or R and X is a random amino acid), and the random peptide libraries were investigated for the acetylcholine receptor inhibitory effect. The random libraries were verified to have acetylcholine receptor binding affinity similar or superior to that of Spep-2.

Hereinafter, an example will be described in which random peptide libraries of 8-mer; (K/R)X(K/R)(K/R)XXX(K/R), 11-mer; XXX(K/R)X(K/R)(K/R)XXX(K/R), 14-mer; XXX(K/R)X(K/R)(K/R)XXX(K/R)XXX, and 18-mer; XXXXX(K/R)X(K/R)(K/R)XXX(K/R)XXXXX ((K/R) is one of either K or R and X is a random amino acid) as representative peptides having excellent binding affinity to acetylcholine receptors was constructed and the acetylcholine receptor inhibitory effect was investigated.

To construct the peptide libraries of 8-mer; (K/R)X(K/R)(K/R)XXX(K/R), 11-mer; XXX(K/R)X(K/R)(K/R)XXX(K/R), 14-mer; XXX(K/R)X(K/R)(K/R)XXX(K/R)XXX, and 18-mer; XXXXX(K/R)X(K/R)(K/R)XXX(K/R)XXXXX ((K/R) is one of either K or R and X is a random amino acid), DNA libraries in Table 5 were synthesized (Bioneer, Korea). Thereafter, PCR was performed using two single-stranded primers (TTCTATGCGGCCCAG and AACAGTTTCTGCGGC) with the synthesized DNA libraries in Table 5 as templates, thereby amplifying insert DNA for insertion of double strands. The amplified insert DNA and the phagemid vector pIGT were digested with the restriction enzymes Sfi I and Not I, and each DNA was purified. The purified insert DNA and pIGT were ligated by T4 DNA ligase, and then precipitates obtained by ethanol precipitation were dissolved in Tris-EDAT (TE) buffer to prepare random peptide library DNA.

The prepared random peptide library DNA was added to and mixed with competent cells, and transformed using electroporation. The transformed cells were placed in a Luria Bertani (LB) liquid medium containing 20 mM glucose, transferred into a test tube, and then cultured at 37°C and 200 rpm for 1 hour. The cultured cells were placed in an LB liquid medium containing 20 mM glucose and 50 $\mu$g/ml ampicillin, and cultured at 30°C for one day. After the one-day culture, the medium was centrifuged at 4°C and 4,000×g for 20 minutes to remove supernatant, thereby securing precipitated cells. The secured cells were suspended in an LB liquid medium, followed by the addition of glycerol to a final concentration of 20% or more, and then stored at -80°C, thereby securing random peptide libraries.

TABLE 5

| Library | Amino acid sequence | Nucleotide sequence |
|---|---|---|
| 8-mer (L1) | (K or R)-X-(K or R) (K or R)-XXX-(K or R), X=random amino acids | TTCTATGCGGCCCAGCTGGCCA<u>RANNKA RAARANNKNNKNNKARA</u>GCGGCCGCAG AAACTGTT |
| 11-mer (L2) | XXX (K or R) -X- (K or R) (K or R) -XXX- (K or R), X=random amino acids | TTCTATGCGGCCCAGCTGGCC<u>NNKNNKN NKARANNKARAARANNKNNKNNKARA</u> GCGGCCGCAGAAACTGTT |
| 14-mer (L3) | XXX (K or R)-X-(K or R) (K or R)-XXX-(K or R) XXX, X=random amino acids | TTCTATGCGGCCCAGCTGGCC<u>NNKNNKN NKARANNKARAARANNKNNKNNKARA NNKNNKNNK</u>GCGGCCGCAGAAACTGTT |
| 18-mer | XXXXX(K or R)-X-(K or R) (K or R)-XXX-(K or R)XXXXX, X=random amino acids | TTCTATGCGGCCCAGCTGGCC<u>NNKNNKN NKNNKNNKARANNKARAARANNKNNK NNKARANNKNNKNNKNNKNNK</u>GCGGC CGCAGAAACTGTT |
| N : A or C or G or T / K : G or T / R : A or G / X : Random amino acids | | |

Example 12-2: Optimized peptide recombinant phage production

The random peptide libraries secured in Example 12-1 were added to an SB liquid medium (3% tryptone, 2% yeast extract, 1% MOPS free acid, and 2% glucose), followed by culture at 37°C and 200 rpm for 20 minutes, and then 1×10$^{10}$ pfu helper phages and ampicillin with a final concentration of 50 $\mu$g/ml were added, followed by culture in the same conditions for 1 hour. The culture was transferred to an SB liquid medium containing 50 $\mu$g/ml ampicillin and 10 $\mu$g/ml kanamycin, and cultured in the same conditions for 16 hours, thereby producing random peptide recombinant phages.

The produced random peptide recombinant phages were centrifuged at 4°C and 5,000 rpm for 10 minutes to obtain supernatant, and the supernatant and polyethylene glycol (PEG)/NaCl (20% PEG, 15% NaCl) were mixed at 5:1 (v:v), left on ice for 1 hour, and centrifuged at 4°C and 13,000 rpm for 20 minutes to remove supernatant. The precipitates were suspended in 1 ml of phosphate buffered saline (PBS) to secure random peptide recombinant phages.

<Example 13: Screening optimized peptides with enhanced binding affinity to acetylcholine receptors>

Example 13-1: Biopanning

A procedure in which immobilized antigens were treated with a phage library surface-expressing antibodies to thereby antibody candidates binding to the antigens is called biopanning, and the biopanning is composed of three steps, binding/washing/elution. The phages having antibodies with weak binding affinity were removed during a washing step, and resultantly, only phages expressing antibodies with strong binding affinity remained. This procedure can be repeated three to four times to discover antibody candidates with excellent antigen binding affinity and specificity. Therefore, biopanning was used to screen acetylcholine receptor-binding peptides with excellent binding affinity and specificity to acetylcholine receptors.

In eight wells of a 96-well plate, 10 $\mu$g/ml AchR $\alpha$1 was placed at 50 $\mu$l per well, and then left at 4°C overnight. The next day, the wells were washed once with 200 ul of Tris (20 mM, pH 7), followed by the addition of 200 ul of 2% bovine serum albumin (BSA), and then blocked at room temperature for 2 hours. Then, the solution was all discarded, and the wells were washed three times with 200 ul of Tris (20 mM, pH 7). After 400 $\mu$l of the random peptide recombinant phages (input phages) suspended in PBS in Example 12-2 was mixed with 400 ul of 2% BSA, the mixture was placed at 100 $\mu$l per well and then left at 30°C for 1 hour. The solution in the wells was all removed, and the wells were washed three times with Tris (20 mM, pH 7). Thereafter, 0.2 M glycine (pH 2.2) was placed at 100 $\mu$l per well, and the phages were isolated for 20 minutes, and then, the phages were collected in one e-tube, and neutralized by the addition of 200$\mu$l of 1 M Tris (pH 9.0) (output phages).

To repeat biopanning, 500 $\mu$l of the isolated phages were mixed with 5 ml of *E. coli,* followed by culture at 37°C and 200 rpm for 30 minutes, and then $1\times10^{10}$ pfu helper phages and ampicillin were added to a final concentration of 50 $\mu$g/ml, followed by further culture for 30 minutes. The culture was transferred to an SB liquid medium containing 50 $\mu$g/ml ampicillin and 10 $\mu$g/ml kanamycin, and cultured in the same conditions overnight, thereby producing random peptide recombinant phages. The reproduced random peptide recombinant phages were centrifuged at 4°C and 5,000 rpm for 10 minutes to obtain supernatant, and then, the supernatant and PEG/NaCl were mixed at 5:1 (v:v), left on ice for 1 hour, and centrifuged at 4°C and 13,000 rpm for 20 minutes to remove supernatant. The precipitates were suspended in 1 ml of phosphate buffered saline (PBS), and used for a second round of biopanning.

Random recombinant phages were reproduced by the same method as above for each round of biopanning, which was performed by the same method as above. In the biopanning steps, the number of times of washing with Tris (20 mM, pH 7) was 3 or 6 times, and biopanning was performed 5 times on AchR.

To measure the number of input phages and output phages for each biopanning, the phages were mixed with *E. coli* having an absorbance at 600 nm of 0.7 (OD600 = 0.7), and plated on agar plates containing ampicillin. The results are shown in Tables 6 to 9 below.

TABLE 6

| 8-mer Biopanning | | | | |
|---|---|---|---|---|
| Round | Number of times of washing | Input phages | Output phages | Input/Output |
| 1st | 3 times | $10.4 \times 10^{11}$ | $264 \times 10^6$ | $25.4\times10^{-5}$ |
| 2nd | 3 times | $2.752 \times 10^{11}$ | $82 \times 10^6$ | $29.8\times10^{-5}$ |
| 3rd | 6 times | $1.024 \times 10^{11}$ | $42.8 \times 10^6$ | $41.8\times10^{-5}$ |
| 4th | 6 times | $15.8 \times 10^{11}$ | $144 \times 10^6$ | $9.11\times10^{-5}$ |
| 5th | 6 times | $12.96 \times 10^{11}$ | $44.8 \times 10^6$ | $3.46\times10^{-5}$ |

TABLE 7

| 11-mer Biopanning | | | | |
|---|---|---|---|---|
| Round | Number of times of washing | Input phages | Output phages | Input/Output |
| 1st | 3 times | $1.14\times10^{12}$ | $1.048\times10^8$ | $8.77\times10^{-5}$ |
| 2nd | 6 times | $9.28\times10^{11}$ | $3.4\times10^6$ | $0.366\times10^{-5}$ |
| 3rd | 6 times | $9.4\times10^{11}$ | $6.96\times10^7$ | $7.4\times10^{-5}$ |

(continued)

| 11-mer Biopanning | | | | |
|---|---|---|---|---|
| Round | Number of times of washing | Input phages | Output phages | Input/Output |
| 4th | 6 times | $1.02\times10^{11}$ | $2.32\times10^7$ | $22.74\times10^{-5}$ |

TABLE 8

| 14-mer Biopanning | | | | |
|---|---|---|---|---|
| Round | Number of times of washing | Input phages | Output phages | Input/Output |
| 1st | 3 times | $1.52 \times 10^{11}$ | $112 \times10^6$ | $73.68 \times10^{-5}$ |
| 2nd | 3 times | $2.112 \times10^{11}$ | $86 \times10^6$ | $40.72 \times10^{-5}$ |
| 3rd | 6 times | $1.0 \times10^{11}$ | $32 \times10^6$ | $32 \times10^{-5}$ |
| 4th | 6 times | $12.7 \times10^{11}$ | $192 \times10^6$ | $15.12 \times10^{-5}$ |
| 5th | 6 times | $17.54 \times10^{11}$ | $29\times10^6$ | $1.65\times10^{-5}$ |

TABLE 9

| 18-mer Biopanning | | | | |
|---|---|---|---|---|
| Round | Number of times of washing | Input phages | Output phages | Input/Output |
| 1st | 3 times | $1.28 \times 10^{12}$ | $5.36 \times10^8$ | $41.87\times10^{-5}$ |
| 2nd | 3 times | $1.6 \times 10^{12}$ | $6.4 \times10^8$ | $40\times10^{-5}$ |
| 3rd | 6 times | $1.58 \times 10^{12}$ | $3.92 \times10^7$ | $2.48\times10^{-5}$ |
| 4th | 6 times | $1.49 \times 10^{11}$ | $9.9 \times10^6$ | $6.6\times10^{-5}$ |
| 5th | 6 times | $1.18 \times 10^{12}$ | $1.16 \times10^8$ | $9.83\times10^{-5}$ |

Example 13-2: Enzyme-linked immunosorbent assay (ELISA) using random peptide library input recombinant phages

ELISA was performed on streptavidin and AchR using the input phages for each round of biopanning in Example 13-1.

In a 96-well ELISA plate, 10 μg/ml AchR or streptavidin was added at 50 μl per well, and left at 4°C overnight. The next day, the wells were washed three times with Tris (20 mM, pH7), followed by the addition of 2% BSA diluted with PBS, and then blocked at room temperature for 2 hours. After the solution was discarded, the wells were washed three times with Tris (20mM, pH7). After 800 μl of the input pages for each of 1st, 2nd, 3rd, 4th, and 5th rounds in Tables 6 to 9 in Example 13-1 were mixed with 200 μl of 10% BSA, the mixture was placed at 100 μl per well and then left at 30°C for 1 hour. The solution in the wells was all removed, and the wells were washed three times with Tris (20 mM, pH 7). Then, horseradish peroxidase (HRP)-conjugated anti-M13 Ab (GE Healthcare) diluted 1:1,000 was added at 100 μl per well, followed by culture at 30°C for 1 hour. After the wells were washed three times with Tris (20 mM, pH 7), 100 μl of a tetramethylbenzidine (TMB) solution, which is a substrate of HRP, was added to each well to induce a color development reaction, and then the reaction was stopped by the addition of 100 μl of 1M HCl, and the absorbance (OD450) was measured at 450 nm. The results are shown in FIG. 13.

As shown in FIG. 13, the 8-mer, 11-mer, and 14-mer random peptide libraries obtained from the input phages of biopanning generally showed very high binding specificity to AchR compared to streptavidin. That is, the library of polypeptides, set forth in the general formula: (K or R)-X-(K or R)-(K or R)-XXX-(K or R), in which the 1st, 3rd, 4th, and 8th amino acids are fixed to be lysine (K) or arginine (R) and the 2nd, 5th, 6th, and 7th amino acids each are an arbitrary amino acid, generally bound to AchR with high specificity, regardless of the types of the 2nd, 5th, 6th, and 7th amino acids (X).

Also, as for 11-Mer, the library of polypeptides, set forth in the general formula: XXX-(K or R)-X-(K or R)-(K or R)-XXX-(K or R) in which the 4th, 6th, 7th, and 11th amino acids are lysine (K) or arginine (R), generally bound to AchR with high specificity, regardless of the type of arbitrary amino acid (1st, 2nd, 3rd, 5th, 8th, 9th and 10th amino acids) expressed by X.

Also, as for 14-Mer, the library of polypeptides, set forth in the general formula: XXX-(K or R)-X-(K or R)-XXX-(K or R)-XXX in which the 4th, 6th, 7th, and 11th amino acids are lysine (K) or arginine (R), generally bound to AchR with high specificity, regardless of the type of arbitrary amino acids (1st, 2nd, 3rd, 5th, 8th, 9th, 10th, 12th, 13th and 14th amino acids) expressed by X.

It was verified through the above results that the acetylcholine receptor-binding peptides according to the present invention containing amino acid sequences set forth in [Formula 1], [Formula 2], [Formula 3], or [Formula 3] bound to AchR with high specificity:

[Formula 1]

$$X_L-(K \text{ or } R)-X-(K \text{ or } R)-X_M-(K \text{ or } R)-X_N$$

($X_L$, $X_M$, and $X_N$ each independently represent a sequence composed of one to eight arbitrary amino acids and X represents a sequence composed of one arbitrary amino acid);

[Formula 2]

$$(K \text{ or } R)-X-(K \text{ or } R)-(K \text{ or } R)-X_{M-1}-(K \text{ or } R)$$

($X_{M-1}$ represents a sequence composed of one to three arbitrary amino acids and X represents a sequence composed of one arbitrary amino acid);

[Formula 3]

$$X_L-(K \text{ or } R)-X-(K \text{ or } R)-(K \text{ or } R)-X_{M-1}-(K \text{ or } R)$$

($X_L$ and $X_{M-1}$ each independently represent a sequence composed of one to three arbitrary amino acids and X represents a sequence composed of one arbitrary amino acid); and

[Formula 4]

$$X_L-(K \text{ or } R)-X-(K \text{ or } R)-(K \text{ or } R)-X_{M-1}-(K \text{ or } R)-X_N$$

($X_L$, $X_{M-1}$ and $X_N$ each independently represent a sequence composed of one to three arbitrary amino acids and X represents a sequence composed of one arbitrary amino acid).

The amino acid sequences were set forth in predetermined formulas on the basis of the crucial sites and sequences of the peptides, and library of the peptides was constructed. The constructed library was verified to bind to acetylcholine receptors to inhibit the actions of the acetylcholine receptors.

As shown in FIG. 13, the 8-mer, 11-mer, and 14-mer random peptide libraries obtained from the input phages of the first round of biopanning showed higher binding specificity to AchR compared with streptavidin, whereas the 18-mer random peptide library obtained from the input phages of the first round of biopanning had low binding affinity to AchR.

It was verified through the above results that the 8-mer to 14-mer random peptides maintained the binding affinity to AchR, but the 18-mer random peptides had reduced binding affinity to AchR. This difference in binding affinity to AchR is considered to be made by the number of random amino acids (X).

Example 13-3: Screening optimized peptides with enhanced binding affinity to acetylcholine receptors

Screening was conducted to select optimized peptides with enhanced AchR binding affinity from the input phages for the 4th and 5th rounds of biopanning, which were identified to have high binding affinity and specificity to AchR in Examples 13-1 and 13-2.

The output phages for the 4th and 5th rounds of biopanning in Example 13-1 were inoculated to *E. coli,* and plated on the agar plates so as to form 100-200 plaques per plate. The plaques were inoculated on 1 ml of an SB liquid medium containing 50 ug/ml of ampicillin by using a sterile tip, and cultured with shaking at 37°C for 5 hours, followed by the addition of 30 µl of helper phages, and then cultured at 37°C and 200 rpm for one day. The culture was centrifuged at 12,000 rpm for 2 minutes to collect supernatant, and 2% BSA was added to the supernatant, and used for phage screening.

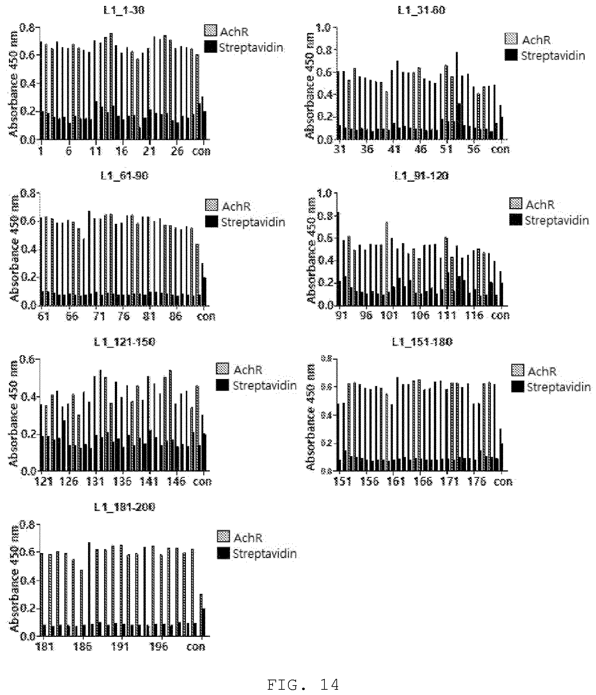

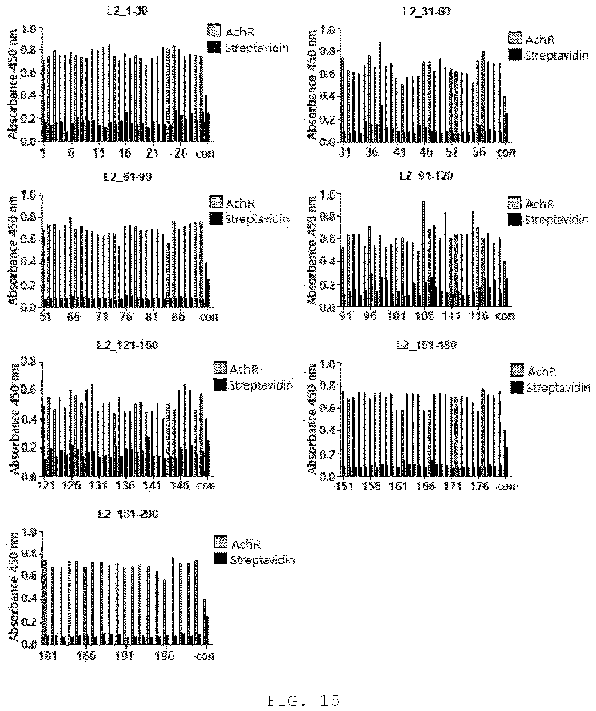

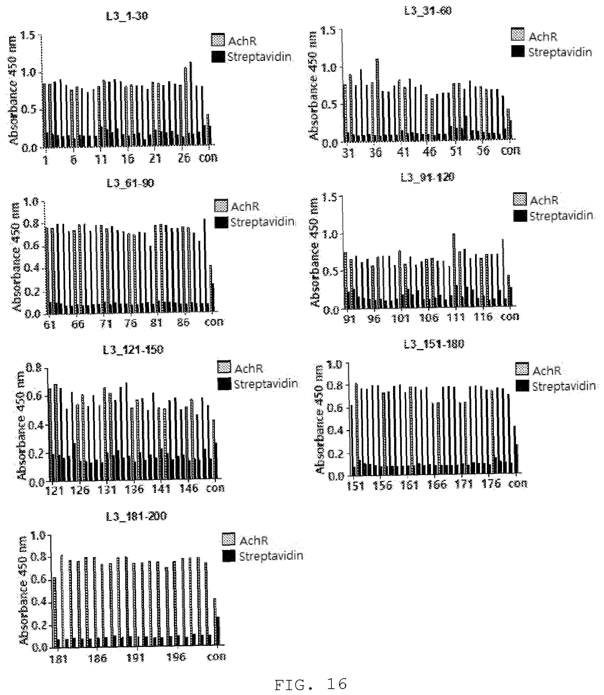

In a 96-well ELISA plate, 5 µg/ml AchR or streptavidin was added at 50 µl per well, and left at 4°C overnight. The next day, the solution in each well was discarded, followed by the addition of 2% BSA, and then blocked at room temperature for 2 hours. After the solution was all discarded, the wells were washed three times with Tris (20mM, pH7). In addition, 100 µl of each of the phage solutions obtained from the above-prepared plaques was dispensed into wells and left at 30°C for 1 hour. After the solution in the wells was all discarded, the wells were washed three times with Tris (20 mM, pH 7), and then the HRP-conjugated anti-M13 antibody diluted 1:2,000 was added at 100 µl per well, followed by culture at 30°C for 1 hour. After the wells were washed three times with Tris (20 mM, pH 7), 100 µl of tetramethylbenzidine was added to each well to induce a color development reaction, and then the reaction was stopped by the addition of 100 µl of 1M $H_2SO_4$, and the absorbance at 450 nm (OD450) was measured. The measured absorbance was converted into a ratio of AchR absorbance to streptavidin absorbance (AchR signal/streptavidin signal), and the results are shown in FIGS. 14 to 16.

As shown in FIGS. 14 to 16, the binding affinity to AchR was observed to be different according to the phage, and among these, phages having the highest AchR signal/streptavidin signal were selected. The results are shown in Tables 10 to 12. The 8-mer (L1), 11-mer (L2), and 14-mer (L3) optimized peptides all showed higher binding affinity and specificity to AchR than Spep-2, a positive control.

Plasmid DNA was purified from the phages selected as above, and peptide sequencing was requested using the purified plasmid DNA and a primer for nucleotide sequencing, composed of GATTACGCCAAGCTTTGGAGC, and the optimized peptides having identified sequences and enhanced binding affinity to AchR were selected.

To compare with the peptides of the amino acid sequences disclosed in Korean Patent Publication No. 10-2014-0139010, the peptides of the amino acid sequences shown in Table 13 were synthesized, and investigated for the binding specificity to AchR by the same method as above. As a result, the synthesized peptides were verified to have the binding specificity similar to that of Spep-2. However, the sequences with R repeated, or the sequences with G repeated at both termini of the 8-mer showed a low level of binding specificity compared with all of the 11-mer or 14-mer library. This is thought to result from the charges or conformational characteristics of the peptides.

TABLE 10

| SEQ ID NO | Peptide (8-mer) | OD | Seq. |
|---|---|---|---|
| 1 | L1-1 | 0.693 | **K**AK**K**IRQ**R** |
| 2 | L1-2 | 0.68 | **K**K**R**K**GSAK** |
| 3 | L1-3 | 0.649 | **R**G**KR**QLG**R** |
| 4 | L1-4 | 0.696 | **K**L**KK**FPV**R** |
| 5 | L1-5 | 0.657 | **R**H**KK**SPW**K** |
| 6 | L1-6 | 0.652 | **R**P**RR**THI**K** |
| 7 | L1-7 | 0.68 | **K**H**KK**LNQ**R** |
| 8 | L1-8 | 0.653 | **R**A**KR**MCC**K** |
| 9 | L1-9 | 0.639 | **R**Q**RR**NDM**K** |
| 10 | L1-10 | 0.621 | **K**R**KK**DWW**R** |
| 11 | L1-11 | 0.703 | **R**A**RK**DAF**K** |
| 12 | L1-12 | 0.691 | **K**W**RR**EGF**K** |
| 13 | L1-13 | 0.73 | **R**F**RR**GVR**R** |
| 14 | L1-14 | 0.755 | **K**A**RR**QED**K** |
| 15 | L1-15 | 0.67 | **R**F**KR**LCM**K** |
| 16 | L1-16 | 0.62 | **K**L**KR**RSR**R** |
| 17 | L1-17 | 0.653 | **R**P**RR**SLD**R** |
| 18 | L1-18 | 0.627 | **K**H**KK**QNV**R** |
| 19 | L1-19 | 0.575 | **K**F**KR**TEG**R** |
| 20 | L1-20 | 0.619 | **R**G**KK**AVR**K** |

(continued)

| SEQ ID NO | Peptide (8-mer) | OD | Seq. |
|---|---|---|---|
| 21 | L1-21 | 0.65 | **KFKKHGVK** |
| 22 | L1-22 | 0.732 | **KTKKIHSK** |
| 23 | L1-23 | 0.712 | **RDRKIHDK** |
| 24 | L1-24 | 0.742 | **RPRRGHQK** |
| 25 | L1-25 | 0.709 | **KTKKLYEK** |
| 26 | L1-26 | 0.647 | **RQKKDNQK** |
| 27 | L1-27 | 0.662 | **RQRKSMQR** |
| 28 | L1-28 | 0.656 | **RGRRWFVK** |
| 29 | L1-29 | 0.646 | **RNKRDENK** |
| 30 | L1-30 | 0.605 | **RLRRWCVK** |
| 31 | L1-31 | 0.604 | **RQKKPWVK** |
| 32 | L1-32 | 0.607 | **RWKRSEQR** |
| 33 | L1-33 | 0.529 | **RLKRAPGR** |
| 34 | L1-34 | 0.634 | **KMRRAQGR** |
| 35 | L1-35 | 0.556 | **KIKKAARR** |
| 36 | L1-36 | 0.553 | **KNRKNLYR** |
| 37 | L1-37 | 0.524 | **KCKKMTER** |
| 38 | L1-38 | 0.514 | **KTRRVDLR** |
| 39 | L1-39 | 0.509 | **KSRKWGWR** |
| 40 | L1-40 | 0.424 | **KRRRFRWK** |
| 41 | L1-41 | 0.613 | **KYKKQHTK** |
| 42 | L1-42 | 0.695 | **RGRKKCGK** |
| 43 | L1-43 | 0.601 | **KLRKQKLR** |
| 44 | L1-44 | 0.59 | **RNRKIPLK** |
| 45 | L1-45 | 0.597 | **RLRRRYRR** |
| 46 | L1-46 | 0.637 | **KWRKRRIK** |
| 47 | L1-47 | 0.54 | **KTKRMLAK** |
| 48 | L1-48 | 0.519 | **KEKRAVHK** |
| 49 | L1-49 | 0.504 | **RVRRDGHR** |
| 50 | L1-50 | 0.583 | **RAKKCYHK** |
| 51 | L1-51 | 0.66 | **KGKRDSNK** |
| 52 | L1-52 | 0.56 | **KVRKSLWK** |
| 53 | L1-53 | 0.78 | **RSKKSLYR** |
| 54 | L1-54 | 0.569 | **RGKRGCER** |
| 55 | L1-55 | 0.585 | **KQKKTGFR** |
| 56 | L1-56 | 0.468 | **KYRRNLVR** |
| 57 | L1-57 | 0.408 | **RIRRGSVR** |
| 58 | L1-58 | 0.472 | **RSKRMNGK** |

(continued)

| SEQ ID NO | Peptide (8-mer) | OD | Seq. |
|---|---|---|---|
| 59 | L1-59 | 0.479 | **R**H**KK**FIE**K** |
| 60 | L1-60 | 0.484 | **RIRR**VSQ**R** |
| 61 | L1-61 | 0.625 | **RPKK**WMQ**K** |
| 62 | L1-62 | 0.633 | **KERK**HEA**K** |
| 63 | L1-63 | 0.618 | **KERK**ASA**K** |
| 64 | L1-64 | 0.589 | **KMRR**IMY**K** |
| 65 | L1-65 | 0.585 | **KWKR**NCT**K** |
| 66 | L1-66 | 0.605 | **RCKK**NPE**K** |
| 67 | L1-67 | 0.593 | **KQRK**PHG**R** |
| 68 | L1-68 | 0.55 | **KAKR**GHF**K** |
| 69 | L1-69 | 0.474 | **KSKR**HHE**K** |
| 70 | L1-70 | 0.668 | **KSRK**HCN**R** |
| 71 | L1-71 | 0.618 | **RSKK**INN**K** |
| 72 | L1-72 | 0.616 | **RVRR**SWI**R** |
| 73 | L1-73 | 0.644 | **KSRR**SAG**K** |
| 74 | L1-74 | 0.649 | **K**MR**K**HDV**R** |
| 75 | L1-75 | 0.581 | **KGKK**LSQ**R** |
| 76 | L1-76 | 0.588 | **KWRR**EQF**R** |
| 77 | L1-77 | 0.636 | **KDKR**WAN**R** |
| 78 | L1-78 | 0.642 | **KHKR**TAQ**R** |
| 79 | L1-79 | 0.583 | **KPKR**EMH**K** |
| 80 | L1-80 | 0.631 | **RWKK**NIN**K** |
| 81 | L1-81 | 0.63 | **KWKK**MME**R** |
| 82 | L1-82 | 0.596 | **RIRR**WWV**R** |
| 83 | L1-83 | 0.621 | **RIRR**HMY**R** |
| 84 | L1-84 | 0.576 | **KPKK**PPA**R** |
| 85 | L1-85 | 0.57 | **RLKR**GQF**K** |
| 86 | L1-86 | 0.551 | **KNKR**TPV**K** |
| 87 | L1-87 | 0.538 | **KWRR**AFG**R** |
| 88 | L1-88 | 0.562 | **KNKK**CVG**R** |
| 89 | L1-89 | 0.551 | **RTRK**CVL**K** |
| 90 | L1-90 | 0.439 | **K**I**KR**DLY**K** |
| 91 | L1-91 | 0.826 | **REKR**YMA**R** |
| 92 | L1-92 | 0.581 | **RVKK**AET**R** |
| 93 | L1-93 | 0.616 | **RGRR**GTM**R** |
| 94 | L1-94 | 0.496 | **RTKR**WQL**K** |
| 95 | L1-95 | 0.535 | **RLRK**SLG**K** |
| 96 | L1-96 | 0.494 | **RPKR**NWA**K** |

(continued)

| SEQ ID NO | Peptide (8-mer) | OD | Seq. |
|---|---|---|---|
| 97 | L1-97 | 0.546 | **KDRK**VNS**R** |
| 98 | L1-98 | 0.54 | **KWRR**NGQ**R** |
| 99 | L1-99 | 0.543 | **RFKR**MVW**R** |
| 100 | L1-100 | 0.737 | **KERK**GAV**R** |
| 101 | L1-101 | 0.599 | **KTKR**GSL**K** |
| 102 | L1-102 | 0.507 | **KTKR**VDP**K** |
| 103 | L1-103 | 0.55 | **RARK**LHN**R** |
| 104 | L1-104 | 0.46 | **RGKK**PWW**R** |
| 105 | L1-105 | 0.507 | **KGKK**YYQ**K** |
| 106 | L1-106 | 0.417 | **RWRR**SVI**K** |
| 107 | L1-107 | 0.534 | **RQKK**DFL**K** |
| 108 | L1-108 | 0.535 | **RGRK**PIW**K** |
| 109 | L1-109 | 0.542 | **KPRR**PDT**R** |
| 110 | L1-110 | 0.422 | **KVKR**LWN**R** |
| 111 | L1-111 | 0.608 | **KDRK**GVY**K** |
| 112 | L1-112 | 0.431 | **RVKR**FVP**K** |
| 113 | L1-113 | 0.526 | **KPKR**DQS**R** |
| 114 | L1-114 | 0.42 | **KDKK**QWG**R** |
| 115 | L1-115 | 0.451 | **RFRK**MIP**K** |
| 116 | L1-116 | 0.492 | **KFRK**HVC**K** |
| 117 | L1-117 | 0.508 | **RPKR**SPS**K** |
| 118 | L1-118 | 0.471 | **RYKK**VPA**K** |
| 119 | L1-119 | 0.463 | **RSRR**CPV**K** |
| 120 | L1-120 | 0.388 | **RCKR**CDN**K** |
| 121 | L1-121 | 0.354 | **RGKK**QLS**K** |
| 122 | L1-122 | 0.352 | **KSKR**PEG**R** |
| 123 | L1-123 | 0.409 | **KCRK**TMG**R** |
| 124 | L1-124 | 0.428 | **RDRK**NPV**R** |
| 125 | L1-125 | 0.345 | **KPKR**SAP**R** |
| 126 | L1-126 | 0.362 | **KGKR**TGC**K** |
| 127 | L1-127 | 0.412 | **RFRK**PTD**R** |
| 128 | L1-128 | 0.301 | **RMKK**FHT**R** |
| 129 | L1-129 | 0.421 | **KIKK**YYW**K** |
| 130 | L1-130 | 0.368 | **RIKR**NNC**K** |
| 131 | L1-131 | 0.505 | **RHKR**MLW**R** |
| 132 | L1-132 | 0.541 | **KVRR**VFL**K** |
| 133 | L1-133 | 0.504 | **KDKR**PEC**K** |
| 134 | L1-134 | 0.365 | **KTRR**SAC**R** |

(continued)

| SEQ ID NO | Peptide (8-mer) | OD | Seq. |
|---|---|---|---|
| 135 | L1-135 | 0.479 | **K**FR**K**GPY**R** |
| 136 | L1-136 | 0.392 | **K**F**KK**SAP**R** |
| 137 | L1-137 | 0.458 | **K**PR**K**IQG**R** |
| 138 | L1-138 | 0.373 | **K**MR**K**GWD**K** |
| 139 | L1-139 | 0.456 | **K**FR**R**QST**R** |
| 140 | L1-140 | 0.381 | **K**NR**R**ADC**R** |
| 141 | L1-141 | 0.505 | **K**NK**R**WVF**K** |
| 142 | L1-142 | 0.469 | **R**CR**K**DTA**R** |
| 143 | L1-143 | 0.416 | **K**AR**R**VGT**K** |
| 144 | L1-144 | 0.503 | **K**V**KK**IYY**R** |
| 145 | L1-145 | 0.54 | **K**AK**R**DYL**R** |
| 146 | L1-146 | 0.362 | **K**VK**R**LPY**R** |
| 147 | L1-147 | 0.415 | **R**HR**R**ANF**R** |
| 148 | L1-148 | 0.426 | **K**P**KK**GGW**K** |
| 149 | L1-149 | 0.34 | **K**EK**R**LYA**R** |
| 150 | L1-150 | 0.457 | **R**E**KK**QEV**K** |
| 151 | L1-151 | 0.479 | **K**SR**R**AVF**R** |
| 152 | L1-152 | 0.484 | **R**HR**K**YVD**K** |
| 153 | L1-153 | 0.625 | **R**HK**R**WSG**R** |
| 154 | L1-154 | 0.633 | **R**DR**K**FVQ**K** |
| 155 | L1-155 | 0.618 | **R**YR**R**WFY**R** |
| 156 | L1-156 | 0.589 | **R**QR**R**MTP**R** |
| 157 | L1-157 | 0.585 | **R**NK**R**MDG**K** |
| 158 | L1-158 | 0.605 | **R**P**KK**HQE**R** |
| 159 | L1-159 | 0.593 | **K**K**RK**SLL**R** |
| 160 | L1-160 | 0.55 | **K**IK**R**NVF**K** |
| 161 | L1-161 | 0.474 | **K**YK**R**EEY**R** |
| 162 | L1-162 | 0.668 | **K**IR**R**VTN**K** |
| 163 | L1-163 | 0.618 | **K**VK**R**VWG**R** |
| 164 | L1-164 | 0.616 | **R**IK**R**DYC**K** |
| 165 | L1-165 | 0.644 | **R**IR**R**AHD**R** |
| 166 | L1-166 | 0.649 | **R**AR**K**ESH**K** |
| 167 | L1-167 | 0.581 | **R**Y**KK**QQY**K** |
| 168 | L1-168 | 0.588 | **K**I**KK**LSQ**K** |
| 169 | L1-169 | 0.636 | **K**LK**R**AML**K** |
| 170 | L1-170 | 0.642 | **K**AR**K**NSI**R** |
| 171 | L1-171 | 0.583 | **K**ER**K**LWW**K** |
| 172 | L1-172 | 0.631 | **K**TR**K**QDH**R** |

(continued)

| SEQ ID NO | Peptide (8-mer) | OD | Seq. |
|---|---|---|---|
| 173 | L1-173 | 0.63 | **RCKR**FVG**K** |
| 174 | L1-174 | 0.596 | **KEKK**LVW**K** |
| 175 | L1-175 | 0.621 | **KARR**NSL**K** |
| 176 | L1-176 | 0.479 | **KMRR**VAP**R** |
| 177 | L1-177 | 0.484 | **RAKK**IMF**R** |
| 178 | L1-178 | 0.625 | **KTKK**AAE**R** |
| 179 | L1-179 | 0.633 | **RGKR**HWH**R** |
| 180 | L1-180 | 0.618 | **RTKK**ENV**K** |
| 181 | L1-181 | 0.589 | **REKK**YAY**K** |
| 182 | L1-182 | 0.585 | **KWRR**ELP**R** |
| 183 | L1-183 | 0.605 | **KSRR**MWG**R** |
| 184 | L1-184 | 0.593 | **KCKK**DND**K** |
| 185 | L1-185 | 0.55 | **KDKK**MPQ**R** |
| 186 | L1-186 | 0.474 | **RHKR**QQD**K** |
| 187 | L1-187 | 0.668 | **RLKK**HCG**K** |
| 188 | L1-188 | 0.618 | **KARK**QEV**R** |
| 189 | L1-189 | 0.616 | **KMRK**FYS**K** |
| 190 | L1-190 | 0.644 | **KMRR**WWL**K** |
| 191 | L1-191 | 0.649 | **KQKR**QWA**R** |
| 192 | L1-192 | 0.581 | **RSRR**GIG**K** |
| 193 | L1-193 | 0.588 | **KDKK**TPC**K** |
| 194 | L1-194 | 0.636 | **RIRK**ITW**R** |
| 195 | L1-195 | 0.642 | **RHKR**WEV**R** |
| 196 | L1-196 | 0.583 | **RFRR**NFH**K** |
| 197 | L1-197 | 0.631 | **KWKR**FSQ**R** |
| 198 | L1-198 | 0.63 | **KYKK**SFT**K** |
| 199 | L1-199 | 0.596 | **KTRK**IVM**K** |
| 200 | L1-200 | 0.621 | **RTKK**AYV**K** |
| | **Spep-2 (con)** | **0.303** | **WTWKGRKSLLR** |

Table 11

| SEQ ID NO | Peptide (11-mer) | OD | Seq. |
|---|---|---|---|
| 201 | L2-1 | 0.705 | EDY**KYRR**QNY**K** |
| 202 | L2-2 | 0.749 | GIW**KFRR**NQC**K** |
| 203 | L2-3 | 0.796 | SPVRW**KR**LCL**R** |
| 204 | L2-4 | 0.757 | ECYR**NRK**AYC**R** |
| 205 | L2-5 | 0.752 | TQQRL**KR**VME**K** |

(continued)

| SEQ ID NO | Peptide (11-mer) | OD | Seq. |
|---|---|---|---|
| 206 | L2-6 | 0.78 | PEL**KC**K**R**MIG**R** |
| 207 | L2-7 | 0.753 | WQM**KTKK**EIW**K** |
| 208 | L2-8 | 0.739 | FGH**RGKK**EWA**R** |
| 209 | L2-9 | 0.721 | ATC**KPKR**PWY**K** |
| 210 | L2-10 | 0.803 | TSQ**KVRK**MEA**K** |
| 211 | L2-11 | 0.791 | GIP**KYRR**GCS**R** |
| 212 | L2-12 | 0.83 | AYA**KARR**WGQ**K** |
| 213 | L2-13 | 0.855 | GGL**KSKR**LTT**R** |
| 214 | L2-14 | 0.746 | GMW**KVRK**TVF**R** |
| 215 | L2-15 | 0.705 | HTT**RGKR**CDP**R** |
| 216 | L2-16 | 0.77 | VDV**RNKK**SNN**R** |
| 217 | L2-17 | 0.72 | GLN**RWRR**CHH**K** |
| 218 | L2-18 | 0.753 | LQS**RSKK**YSV**K** |
| 219 | L2-19 | 0.727 | GWN**RAKR**EES**K** |
| 220 | L2-20 | 0.675 | SWQ**KCKK**AEV**R** |
| 221 | L2-21 | 0.719 | MVM**KWKR**WNQ**R** |
| 222 | L2-22 | 0.75 | MMM**RHKR**CQY**R** |
| 223 | L2-23 | 0.832 | QTY**RLKK**PLQ**K** |
| 224 | L2-24 | 0.812 | SFW**RERK**NGF**R** |
| 225 | L2-25 | 0.842 | RLE**RPRR**ELT**K** |
| 226 | L2-26 | 0.809 | APW**RLKR**APQ**R** |
| 227 | L2-27 | 0.747 | QGE**KYRR**TES**K** |
| 228 | L2-28 | 0.762 | CIW**KSKR**SPA**K** |
| 229 | L2-29 | 0.756 | AIL**KGKR**IPN**K** |
| 230 | L2-30 | 0.746 | PTL**KWRK**PVL**R** |
| 231 | L2-31 | 0.737 | NNS**RSKR**FMN**R** |
| 232 | L2-32 | 0.64 | LYM**RCKK**QAP**R** |
| 233 | L2-33 | 0.619 | INY**RCRK**WVD**K** |
| 234 | L2-34 | 0.604 | VMD**RDRK**WWW**R** |
| 235 | L2-35 | 0.683 | NFT**KLRK**PGP**R** |
| 236 | L2-36 | 0.76 | FIP**KMRK**CSP**R** |
| 237 | L2-37 | 0.66 | TLW**KVRK**AYS**R** |
| 238 | L2-38 | 0.88 | ESE**KIKR**LST**R** |
| 239 | L2-39 | 0.669 | LMT**KNRR**NSF**R** |
| 240 | L2-40 | 0.685 | PEV**KVRR**HSM**R** |
| 241 | L2-41 | 0.568 | AEV**KDKK**AYY**K** |
| 242 | L2-42 | 0.508 | YYA**KSKK**YMV**R** |
| 243 | L2-43 | 0.572 | LMD**RTRR**DMY**K** |

(continued)

| SEQ ID NO | Peptide (11-mer) | OD | Seq. |
|---|---|---|---|
| 244 | L2-44 | 0.579 | NMG**RH**K**R**PFL**K** |
| 245 | L2-45 | 0.584 | WIY**RG**R**K**DVA**K** |
| 246 | L2-46 | 0.704 | CYW**KA**K**R**YPM**R** |
| 247 | L2-47 | 0.707 | IED**KG**R**R**INP**R** |
| 248 | L2-48 | 0.629 | VST**KI**K**K**EPQ**R** |
| 249 | L2-49 | 0.734 | YPF**KA**K**R**PAE**K** |
| 250 | L2-50 | 0.656 | SAD**RN**K**R**HMT**R** |
| 251 | L2-51 | 0.653 | SLY**RQ**K**R**HDY**K** |
| 252 | L2-52 | 0.624 | LPS**RP**K**K**PVP**K** |
| 253 | L2-53 | 0.614 | PAW**RC**K**R**CQP**K** |
| 254 | L2-54 | 0.609 | HHW**RF**K**R**EMP**R** |
| 255 | L2-55 | 0.524 | PAC**RS**K**R**DWQ**K** |
| 256 | L2-56 | 0.713 | FIC**KS**R**K**FYG**K** |
| 257 | L2-57 | 0.795 | LEH**KE**R**K**DDF**R** |
| 258 | L2-58 | 0.701 | ANP**KN**R**K**DNL**R** |
| 259 | L2-59 | 0.69 | FGV**KY**R**R**VIC**R** |
| 260 | L2-60 | 0.697 | HAD**KF**R**R**FNM**R** |
| 261 | L2-61 | 0.688 | PTI**RV**R**K**SDD**R** |
| 262 | L2-62 | 0.736 | NLH**KP**K**R**DLP**K** |
| 263 | L2-63 | 0.742 | IDG**KW**R**K**ICT**R** |
| 264 | L2-64 | 0.683 | SPF**RA**K**R**QDV**R** |
| 265 | L2-65 | 0.731 | PLW**KT**R**K**IEP**R** |
| 266 | L2-66 | 0.798 | PNY**KW**R**K**SRR**R** |
| 267 | L2-67 | 0.696 | KTG**RS**K**R**HRW**R** |
| 268 | L2-68 | 0.721 | GPD**KS**R**R**NLH**R** |
| 269 | L2-69 | 0.676 | WCC**KT**K**R**AVM**K** |
| 270 | L2-70 | 0.67 | QLL**KM**R**K**ALS**R** |
| 271 | L2-71 | 0.651 | PYL**RS**K**R**FPP**R** |
| 272 | L2-72 | 0.638 | APH**KW**R**K**QEQ**R** |
| 273 | L2-73 | 0.662 | PPF**KF**R**K**PLG**R** |
| 274 | L2-74 | 0.651 | CIM**RV**K**R**MWW**K** |
| 275 | L2-75 | 0.539 | YIL**RD**K**K**VPL**K** |
| 276 | L2-76 | 0.725 | PWV**RI**R**K**MAS**R** |
| 277 | L2-77 | 0.733 | VAD**RQ**K**K**TAP**K** |
| 278 | L2-78 | 0.718 | HHN**RM**K**K**QYH**R** |
| 279 | L2-79 | 0.689 | EGA**KY**R**R**DGW**R** |
| 280 | L2-80 | 0.685 | FWD**RV**K**R**NPS**K** |
| 281 | L2-81 | 0.705 | TMWRQRKMSCK |

(continued)

| SEQ ID NO | Peptide (11-mer) | OD | Seq. |
|---|---|---|---|
| 282 | L2-82 | 0.693 | PDTRWKRVLFK |
| 283 | L2-83 | 0.65 | HQQKCKKTTTK |
| 284 | L2-84 | 0.574 | PWHKGKRDFDR |
| 285 | L2-85 | 0.768 | VFVKWRRQMMR |
| 286 | L2-86 | 0.701 | SQWKIRKRLIR |
| 287 | L2-87 | 0.716 | EWHKVRRVYAK |
| 288 | L2-88 | 0.744 | NAMRTKRMSFK |
| 289 | L2-89 | 0.749 | PPFKFRKPLGR |
| 290 | L2-90 | 0.765 | TSVKKRKQRLR |
| 291 | L2-91 | 0.517 | PID**KFKR**GMV**R** |
| 292 | L2-92 | 0.634 | FVG**RWKR**EYA**K** |
| 293 | L2-93 | 0.635 | FNP**KMRK**VCL**R** |
| 294 | L2-94 | 0.642 | NPG**KTKR**WLQ**R** |
| 295 | L2-95 | 0.522 | IMD**KRRK**PGC**R** |
| 296 | L2-96 | 0.708 | ISD**KAKR**QHF**K** |
| 297 | L2-97 | 0.531 | DVN**KYRK**HSH**K** |
| 298 | L2-98 | 0.626 | LML**RGKR**LTT**K** |
| 299 | L2-99 | 0.52 | CSL**RARK**EEW**R** |
| 300 | L2-100 | 0.551 | MNY**RCKR**VQE**R** |
| 301 | L2-101 | 0.592 | NGV**RERK**WQS**K** |
| 302 | L2-102 | 0.608 | YTS**RCKK**QPR**K** |
| 303 | L2-103 | 0.571 | QYN**KGRK**IHV**R** |
| 304 | L2-104 | 0.563 | PED**RQRK**TWF**K** |
| 305 | L2-105 | 0.488 | IEM**KPRK**FGV**K** |
| 306 | L2-106 | 0.926 | QRW**KWRK**SLA**R** |
| 307 | L2-107 | 0.681 | VKH**KERK**CQR**R** |
| 308 | L2-108 | 0.716 | QQD**RPKR**DIP**K** |
| 309 | L2-109 | 0.596 | YPT**KGKR**CMI**R** |
| 310 | L2-110 | 0.831 | RYA**KHRK**RQT**R** |
| 311 | L2-111 | 0.594 | GYF**RPRK**ETC**K** |
| 312 | L2-112 | 0.646 | CFF**KMRR**CNT**K** |
| 313 | L2-113 | 0.64 | QND**KDRK**LSH**R** |
| 314 | L2-114 | 0.643 | QVT**KSKR**VAF**K** |
| 315 | L2-115 | 0.837 | VRA**KHRK**SSL**R** |
| 316 | L2-116 | 0.699 | SHS**RQRK**TPL**R** |
| 317 | L2-117 | 0.607 | DNG**KIRR**CLG**K** |
| 318 | L2-118 | 0.65 | FLR**RLKK**VHW**K** |
| 319 | L2-119 | 0.56 | FCR**RSKK**IGRR |

(continued)

| SEQ ID NO | Peptide (11-mer) | OD | Seq. |
|---|---|---|---|
| 320 | L2-120 | 0.607 | PAARTKRMYGR |
| 321 | L2-121 | 0.492 | HETKPKKDGLR |
| 322 | L2-122 | 0.558 | MGDRPRKWDSR |
| 323 | L2-123 | 0.473 | HDTKCKKMYAK |
| 324 | L2-124 | 0.556 | NVVRGRRLECR |
| 325 | L2-125 | 0.481 | PADKGRREVMK |
| 326 | L2-126 | 0.605 | AMMKYKKEFPK |
| 327 | L2-127 | 0.569 | YIIRWKRQMTR |
| 328 | L2-128 | 0.516 | SPWRIRRQNIR |
| 329 | L2-129 | 0.603 | TCIKYRRAHTK |
| 330 | L2-130 | 0.64 | GYDRARKGTLR |
| 331 | L2-131 | 0.462 | MTLKHRRVYIK |
| 332 | L2-132 | 0.515 | LFTRIKRLVCK |
| 333 | L2-133 | 0.526 | DFSRDRRCLSK |
| 334 | L2-134 | 0.44 | VWNKVKRWLER |
| 335 | L2-135 | 0.557 | FPGKNRKYCSR |
| 336 | L2-136 | 0.454 | YTSKNKRGCPR |
| 337 | L2-137 | 0.452 | CACKQRRATSR |
| 338 | L2-138 | 0.509 | IMERQRKSQHR |
| 339 | L2-139 | 0.528 | SVLRCRKCSMK |
| 340 | L2-140 | 0.445 | YPQKLRRTALK |
| 341 | L2-141 | 0.462 | SSHKGKRAQSK |
| 342 | L2-142 | 0.512 | DNPRFRKTILK |
| 343 | L2-143 | 0.401 | AIIKFRKVQWK |
| 344 | L2-144 | 0.521 | PYTRCRKEICK |
| 345 | L2-145 | 0.468 | MNEKPKKNDQK |
| 346 | L2-146 | 0.605 | LIGKFKKPFYR |
| 347 | L2-147 | 0.641 | TWCKHKKLDMK |
| 348 | L2-148 | 0.604 | DSNKVRKCSSK |
| 349 | L2-149 | 0.465 | LPMKQRKCEFR |
| 350 | L2-150 | 0.579 | EDVRVKRQTCR |
| 351 | L2-151 | 0.749 | TQCKDRRVSDR |
| 352 | L2-152 | 0.681 | IALKPKRVWLK |
| 353 | L2-153 | 0.688 | GHQRGKREGSR |
| 354 | L2-154 | 0.736 | NPFKYKKICPK |
| 355 | L2-155 | 0.742 | NEARIKKCDVK |
| 356 | L2-156 | 0.683 | YGLRMRKWYMK |
| 357 | L2-157 | 0.731 | NFYKCRKLQCK |

(continued)

| SEQ ID NO | Peptide (11-mer) | OD | Seq. |
|---|---|---|---|
| 358 | L2-158 | 0.73 | MMT**KY**K**K**TCC**K** |
| 359 | L2-159 | 0.696 | CNQ**KTKK**IAE**K** |
| 360 | L2-160 | 0.721 | ADI**RMKK**WYP**K** |
| 361 | L2-161 | 0.579 | AWF**RVKR**SNC**R** |
| 362 | L2-162 | 0.584 | NCD**RTRK**HWA**R** |
| 363 | L2-163 | 0.725 | PHA**RTRK**NIT**K** |
| 364 | L2-164 | 0.733 | VPT**KMKK**YET**K** |
| 365 | L2-165 | 0.718 | AYP**KFRK**TFN**R** |
| 366 | L2-166 | 0.579 | QQL**RLRK**LCG**K** |
| 367 | L2-167 | 0.584 | MFM**RNKK**LAW**R** |
| 368 | L2-168 | 0.725 | GGA**KNKK**VVS**R** |
| 369 | L2-169 | 0.733 | HDP**RHKK**TPT**K** |
| 370 | L2-170 | 0.718 | QVH**RNKR**YTD**R** |
| 371 | L2-171 | 0.689 | YGT**RPKK**YVS**K** |
| 372 | L2-172 | 0.685 | CTW**RGRR**PHD**K** |
| 373 | L2-173 | 0.705 | SWA**KARK**LVH**R** |
| 374 | L2-174 | 0.693 | PLF**KSRR**AYV**R** |
| 375 | L2-175 | 0.65 | CVM**RCRR**SED**K** |
| 376 | L2-176 | 0.574 | WWH**KHRR**AQS**K** |
| 377 | L2-177 | 0.768 | LNS**KPRR**VEF**K** |
| 378 | L2-178 | 0.718 | VPY**RHRR**MQF**K** |
| 379 | L2-179 | 0.716 | GIA**KSKR**NAG**R** |
| 380 | L2-180 | 0.744 | DPE**KWRK**FYD**R** |
| 381 | L2-181 | 0.749 | PGA**RCRK**QDV**K** |
| 382 | L2-182 | 0.681 | HEQ**RPKK**GQQ**K** |
| 383 | L2-183 | 0.688 | TCD**RARK**ESF**R** |
| 384 | L2-184 | 0.736 | MHQ**RERR**NFV**K** |
| 385 | L2-185 | 0.742 | FIT**RFRK**MGE**K** |
| 386 | L2-186 | 0.683 | PNW**KVRR**FGD**K** |
| 387 | L2-187 | 0.731 | TAA**KWKK**IIM**K** |
| 388 | L2-188 | 0.73 | CLW**RLRK**DNG**R** |
| 389 | L2-189 | 0.696 | AHI**KSRK**VWS**R** |
| 390 | L2-190 | 0.721 | NLV**KSKK**VEE**K** |
| 391 | L2-191 | 0.689 | PDS**RLKK**HEA**K** |
| 392 | L2-192 | 0.685 | EHV**KLKR**LDF**R** |
| 393 | L2-193 | 0.705 | PAL**RMRR**WCQ**K** |
| 394 | L2-194 | 0.693 | HLE**KHRR**CEF**K** |
| 395 | L2-195 | 0.65 | CQA**KTRK**AED**R** |

(continued)

| SEQ ID NO | Peptide (11-mer) | OD | Seq. |
|---|---|---|---|
| 396 | L2-196 | 0.574 | QNM**R**M**KR**FIQ**R** |
| 397 | L2-197 | 0.768 | CPY**RIRR**GPG**K** |
| 398 | L2-198 | 0.718 | DIY**K**G**KR**TLV**K** |
| 399 | L2-199 | 0.716 | EIC**K**N**RK**PAN**R** |
| 400 | L2-200 | 0.744 | QGM**K**L**KR**IWS**K** |
| | **Spep-2 (con)** | **0.303** | **WTWKGRKSLLR** |

TABLE 12

| SEQ ID NO | Peptide (14-mer) | OD | Seq. |
|---|---|---|---|
| 401 | L3-1 | 0.853 | ALG**R**T**KR**LHM**R**VHV |
| 402 | L3-2 | 0.841 | SFY**K**E**RK**CQF**R**SGL |
| 403 | L3-3 | 0.88 | SGV**R**Y**RK**WWI**R**SW |
| 404 | L3-4 | 0.905 | MPQ**K**L**KK**LDI**R**NHN |
| 405 | L3-5 | 0.82 | NED**R**G**KR**PHI**R**VLG |
| 406 | L3-6 | 0.77 | EFV**K**L**RK**ARL**R**GPQ |
| 407 | L3-7 | 0.803 | EGD**K**F**RR**HDI**K**YNF |
| 408 | L3-8 | 0.777 | PIC**R**W**KR**APF**K**WYF |
| 409 | L3-9 | 0.725 | NCN**R**F**RR**TIV**R**HCH |
| 410 | L3-10 | 0.769 | AHG**K**D**RR**YVE**K**LEV |
| 411 | L3-11 | 0.8 | FMQ**K**C**KK**WWD**R**AVF |
| 412 | L3-12 | 0.882 | DPP**K**K**RK**SLL**R**RVS |
| 413 | L3-13 | 0.862 | YCT**RIRR**EGM**K**GSE |
| 414 | L3-14 | 0.892 | FPG**K**S**KK**QWH**R**LWP |
| 415 | L3-15 | 0.859 | YEP**R**F**KR**PYG**K**WCH |
| 416 | L3-16 | 0.797 | DYL**RS RK**MEE**R**FQE |
| 417 | L3-17 | 0.812 | VGP**K**F**RK**NH**RR**QNR |
| 418 | L3-18 | 0.806 | EYQ**K**C**KK**PSF**R**LTM |
| 419 | L3-19 | 0.796 | YM**K**K**RK**SLL**R**TSL |
| 420 | L3-20 | 0.755 | CCF**K**L**KK**AYN**K**GPF |
| 421 | L3-21 | 0.843 | NPM**K**L**RK**AET**K**HNV |
| 422 | L3-22 | 0.83 | HSL**K**T**RK**SAF**K**SNT |
| 423 | L3-23 | 0.799 | QYH**K**V**RK**LWF**R**VEP |
| 424 | L3-24 | 0.846 | FV**R**K**RK**SLL**R**DTR |
| 425 | L3-25 | 0.807 | CFQ**R**K**RK**SLL**R**VLR |
| 426 | L3-26 | 0.802 | VPM**K**Y**RR**CGN**R**QSN |
| 427 | L3-27 | 0.83 | RA**R**K**RK**SLL**RR**QV |
| 428 | L3-28 | 0.803 | PNG**K**V**RK**IR**RR**YF |

(continued)

| SEQ ID NO | Peptide (14-mer) | OD | Seq. |
|---|---|---|---|
| 429 | L3-29 | 0.789 | CAS**K**P**RR**TYL**R**AAN |
| 430 | L3-30 | 0.771 | WAH**K**C**KK**PGQ**R**IPP |
| 431 | L3-31 | 0.763 | LSD**KK**R**K**SLL**R**YDY |
| 432 | L3-32 | 0.845 | CAT**R**G**KK**VFS**K**RTM |
| 433 | L3-33 | 0.751 | LRQ**R**S**KR**VLE**K**LRP |
| 434 | L3-34 | 0.74 | LGW**KK**R**K**SLL**R**RHV |
| 435 | L3-35 | 0.747 | LQC**K**Y**RR**GSD**K**QPQ |
| 436 | L3-36 | 0.787 | AFS**R**I**KR**GVL**K**LLS |
| 437 | L3-37 | 0.69 | SNV**K**R**KR**GRC**K**PYR |
| 438 | L3-38 | 0.669 | TVV**K**S**RK**CSV**R**YNW |
| 439 | L3-39 | 0.654 | DDV**K**Q**RK**KHP**R**VQT |
| 440 | L3-40 | 0.733 | ASP**K**G**RR**PTF**R**PQH |
| 441 | L3-41 | 0.81 | GWL**K**A**KR**FPS**R**PPT |
| 442 | L3-42 | 0.71 | ETN**R**T**RK**QCY**K**TTF |
| 443 | L3-43 | 0.93 | FTH**K**N**RR**DSL**R**VWM |
| 444 | L3-44 | 0.719 | PPF**R**L**RK**PLW**R**PQR |
| 445 | L3-45 | 0.735 | PGN**R**M**KK**YQN**R**VHG |
| 446 | L3-46 | 0.618 | SAH**KK**R**K**QTL**R**CSE |
| 447 | L3-47 | 0.558 | LYE**K**Y**KK**HNN**R**EDD |
| 448 | L3-48 | 0.622 | PHC**R**Q**KK**FWI**R**CGT |
| 449 | L3-49 | 0.629 | VAF**K**T**RR**VQ**R**QSG |
| 450 | L3-50 | 0.634 | VFD**K**F**RK**TEN**R**GVI |
| 451 | L3-51 | 0.754 | NHH**K**T**KR**CSV**R**FNI |
| 452 | L3-52 | 0.757 | GTF**K**W**RK**SGA**R**QYL |
| 453 | L3-53 | 0.679 | HCL**R**T**KK**LIN**K**ICS |
| 454 | L3-54 | 0.784 | FFL**R**C**RR**LLG**K**VQV |
| 455 | L3-55 | 0.706 | HFP**R**A**RR**FEH**R**CML |
| 456 | L3-56 | 0.703 | QIS**K**L**KR**PSY**R**GDD |
| 457 | L3-57 | 0.674 | EEI**K**Q**KK**LHL**R**VWF |
| 458 | L3-58 | 0.664 | SLP**K**W**RK**GGD**R**VFT |
| 459 | L3-59 | 0.659 | HVY**K**N**RR**VWG**K**GWP |
| 460 | L3-60 | 0.574 | EDL**R**C**KK**LEL**R**SVI |
| 461 | L3-61 | 0.768 | THD**K**C**KK**HND**K**QAH |
| 462 | L3-62 | 0.766 | YPE**R**P**RK**LQD**K**SYS |
| 463 | L3-63 | 0.794 | CPW**R**N**RK**AMI**K**GII |
| 464 | L3-64 | 0.799 | HEI**K**Q**KK**YFH**R**GHD |
| 465 | L3-65 | 0.731 | CLE**K**L**RK**AVH**R**QRR |
| 466 | L3-66 | 0.738 | YIS**K**S**KK**TAG**R**WFW |

(continued)

| SEQ ID NO | Peptide (14-mer) | OD | Seq. |
|---|---|---|---|
| 467 | L3-67 | 0.786 | VTW**KFRK**AEK**R**WGY |
| 468 | L3-68 | 0.792 | YSS**KSRK**LSP**R**TPR |
| 469 | L3-69 | 0.733 | CVN**RVKK**SDS**K**GTW |
| 470 | L3-70 | 0.781 | DHE**RERR**LWS**R**FPF |
| 471 | L3-71 | 0.78 | PLI**KVKR**GVG**K**LWN |
| 472 | L3-72 | 0.746 | TGC**RCKR**SMY**K**NLH |
| 473 | L3-73 | 0.771 | WTC**RMRK**YQL**R**TSE |
| 474 | L3-74 | 0.726 | IAI**RPRK**MTL**K**IHP |
| 475 | L3-75 | 0.72 | QIP**KQKK**QEQ**R**AIS |
| 476 | L3-76 | 0.701 | QMG**KFKK**ISL**K**NTF |
| 477 | L3-77 | 0.688 | VLI**KQRK**WQD**R**SCS |
| 478 | L3-78 | 0.712 | FIT**KSRR**QQF**R**NQG |
| 479 | L3-79 | 0.701 | QQF**KYRR**ECV**K**YGS |
| 480 | L3-80 | 0.589 | MEG**REKK**SYN**K**GEN |
| 481 | L3-81 | 0.775 | PTW**RHRR**YCA**K**DIG |
| 482 | L3-82 | 0.783 | HMC**RSKR**VAW**K**NLL |
| 483 | L3-83 | 0.768 | SYL**KCKR**SDY**K**EVP |
| 484 | L3-84 | 0.739 | HTF**KLRK**LCQ**K**LFE |
| 485 | L3-85 | 0.735 | QLV**KLRK**LAR**R**VSY |
| 486 | L3-86 | 0.755 | SEV**RPKK**DHS**R**LFI |
| 487 | L3-87 | 0.743 | FLE**KCRK**FII**R**VST |
| 488 | L3-88 | 0.7 | PET**RHRK**MFI**R**DFW |
| 489 | L3-89 | 0.624 | EWC**KNKR**WHS**R**EYP |
| 490 | L3-90 | 0.818 | LWN**RYKK**LLM**R**IFW |
| 491 | L3-91 | 0.749 | CWC**RQRK**CFH**K**PWI |
| 492 | L3-92 | 0.657 | NLE**RTKK**HGL**K**GYM |
| 493 | L3-93 | 0.7 | EGG**RIKR**PNY**R**GDG |
| 494 | L3-94 | 0.61 | PVL**KLRK**GRV**R**AQP |
| 495 | L3-95 | 0.657 | SIS**RLRK**AHQ**K**FIP |
| 496 | L3-96 | 0.567 | EWH**KHRR**EMV**R**WGP |
| 497 | L3-97 | 0.684 | WSA**KGRR**MGC**K**STM |
| 498 | L3-98 | 0.685 | PNG**KVRK**RIR**RR**YF |
| 499 | L3-99 | 0.692 | PCW**KFRR**AQM**K**WGI |
| 500 | L3-100 | 0.572 | LEE**RPKK**HCA**K**NHC |
| 501 | L3-101 | 0.758 | QPW**RQKK**FWC**R**CWG |
| 502 | L3-102 | 0.581 | IMV**KIRR**CPA**R**PLC |
| 503 | L3-103 | 0.676 | NDW**KLRK**DFW**R**NHF |
| 504 | L3-104 | 0.57 | ASV**RDRK**MGY**K**SDG |

(continued)

| SEQ ID NO | Peptide (14-mer) | OD | Seq. |
|---|---|---|---|
| 505 | L3-105 | 0.601 | YYE**KIRR**GEI**K**IAE |
| 506 | L3-106 | 0.642 | DLV**RKRK**TML**R**QLV |
| 507 | L3-107 | 0.658 | DLS**KVRR**GHG**K**NDI |
| 508 | L3-108 | 0.621 | ESY**RLRR**GTD**K**EQW |
| 509 | L3-109 | 0.613 | YIT**KFRK**FLM**K**DQF |
| 510 | L3-110 | 0.538 | AWA**KYKK**VQP**K**HHT |
| 511 | L3-111 | 0.976 | TWL**RGRR**WPD**K**QPQ |
| 512 | L3-112 | 0.731 | ALP**RVRK**DSH**R**EEI |
| 513 | L3-113 | 0.766 | CLF**KYRK**SCV**R**LCG |
| 514 | L3-114 | 0.646 | LQN**RMRK**IYW**K**TFD |
| 515 | L3-115 | 0.685 | NDP**RLRK**HNH**R**CCT |
| 516 | L3-116 | 0.644 | YGS**KQRR**FEE**K**ICG |
| 517 | L3-117 | 0.696 | CGH**KNKK**WHN**R**MDP |
| 518 | L3-118 | 0.69 | YGI**RDRR**PMT**K**HTQ |
| 519 | L3-119 | 0.693 | TPL**KSRK**YWN**K**HAY |
| 520 | L3-120 | 0.887 | FLY**KAKK**ALM**R**DSL |
| 521 | L3-121 | 0.655 | CDD**KPKK**TW**K**LTW |
| 522 | L3-122 | 0.691 | MHP**RPRK**MSH**K**MSC |
| 523 | L3-123 | 0.654 | PGE**RLKK**EDH**R**ASG |
| 524 | L3-124 | 0.515 | GAQ**RARK**PVL**R**AVG |
| 525 | L3-125 | 0.629 | MQE**KCRR**CVF**K**GNI |
| 526 | L3-126 | 0.542 | YVD**RWRR**MQY**K**LSI |
| 527 | L3-127 | 0.608 | WVD**RSRK**FED**R**NCL |
| 528 | L3-128 | 0.523 | IVN**RAKR**SQI**R**FDV |
| 529 | L3-129 | 0.606 | IHP**RYKR**HQA**R**SCC |
| 530 | L3-130 | 0.531 | EHS**KHRK**DLF**R**MVG |
| 531 | L3-131 | 0.655 | YTP**KFRR**IFW**R**IID |
| 532 | L3-132 | 0.619 | TIT**RVKR**ASH**K**TPS |
| 533 | L3-133 | 0.566 | VDS**REKK**WRQ**K**CQC |
| 534 | L3-134 | 0.653 | YWF**RIKR**AHA**K**GCP |
| 535 | L3-135 | 0.69 | NDH**RMRR**SVD**R**GET |
| 536 | L3-136 | 0.512 | PFD**RARR**DLHRIMM |
| 537 | L3-137 | 0.565 | QVV**RLRR**GKN**R**GTV |
| 538 | L3-138 | 0.576 | TNQ**RPRR**ETA**K**TIE |
| 539 | L3-139 | 0.49 | FHH**RHKR**IAT**K**HPA |
| 540 | L3-140 | 0.607 | EHC**KWKK**MFD**K**EGE |
| 541 | L3-141 | 0.504 | FFY**RLRR**CLT**R**WWV |
| 542 | L3-142 | 0.502 | LQC**KYRR**GSD**K**TVV |

(continued)

| SEQ ID NO | Peptide (14-mer) | OD | Seq. |
|---|---|---|---|
| 543 | L3-143 | 0.559 | QTM**KF**K**R**CCN**K**EMV |
| 544 | L3-144 | 0.578 | CSY**KLRR**CDM**R**LWG |
| 545 | L3-145 | 0.495 | MHI**KMKR**EQV**K**DEE |
| 546 | L3-146 | 0.512 | AVW**RNRK**DNV**K**ASE |
| 547 | L3-147 | 0.562 | ADH**RAKK**TLS**K**FWT |
| 548 | L3-148 | 0.451 | MMA**RCRR**IMC**R**SFN |
| 549 | L3-149 | 0.571 | PFG**KFKR**LEN**K**DEC |
| 550 | L3-150 | 0.518 | YLS**RTKR**WLA**R**YVE |
| 551 | L3-151 | 0.624 | HTL**KPKR**LYP**R**PSE |
| 552 | L3-152 | 0.818 | VVI**KCRK**WTH**K**MDH |
| 553 | L3-153 | 0.768 | FHT**KLRK**PIP**K**IDM |
| 554 | L3-154 | 0.766 | ENI**KSRK**YFV**K**LTW |
| 555 | L3-155 | 0.794 | AWT**RFRR**FQC**K**GMS |
| 556 | L3-156 | 0.799 | IVP**RDRK**QAL**R**WTN |
| 557 | L3-157 | 0.731 | CML**RWKR**WHM**R**FNP |
| 558 | L3-158 | 0.738 | FDM**RDRR**PNA**R**VMP |
| 559 | L3-159 | 0.786 | QIA**KLKR**DQM**K**EAW |
| 560 | L3-160 | 0.792 | LTS**RCKK**TFQ**K**NSE |
| 561 | L3-161 | 0.733 | MAA**KWKK**DGM**K**SHY |
| 562 | L3-162 | 0.781 | ANW**RTRR**DLA**K**EHV |
| 563 | L3-163 | 0.78 | PFT**KMRK**QFQ**R**MSA |
| 564 | L3-164 | 0.746 | QIN**RVKK**SDA**K**FCT |
| 565 | L3-165 | 0.771 | YLV**KLRR**FWA**K**IDM |
| 566 | L3-166 | 0.629 | EYV**KAKR**TPW**K**YVV |
| 567 | L3-167 | 0.634 | AMC**REKR**FPY**R**AIY |
| 568 | L3-168 | 0.775 | MGL**RARK**YED**K**TLH |
| 569 | L3-169 | 0.783 | FHM**KSKK**DGD**K**FVM |
| 570 | L3-170 | 0.768 | CIF**KFKK**NMF**K**GYS |
| 571 | L3-171 | 0.629 | LDT**KNRR**GAS**K**WDY |
| 572 | L3-172 | 0.634 | VTG**KGRR**VTF**R**CMN |
| 573 | L3-173 | 0.775 | QLG**KQKR**EAT**R**EYV |
| 574 | L3-174 | 0.783 | SSG**KWKR**PHA**K**YIV |
| 575 | L3-175 | 0.768 | TCH**KTKR**SIM**R**ATS |
| 576 | L3-176 | 0.739 | MIG**KCRK**CGT**K**CYA |
| 577 | L3-177 | 0.735 | FQP**KSRR**AET**K**HSY |
| 578 | L3-178 | 0.755 | PDL**KFRK**HQD**R**TAI |
| 579 | L3-179 | 0.743 | YNA**RVKR**EGI**K**NIT |
| 580 | L3-180 | 0.7 | QGI**RPRK**HLQ**R**MPN |

(continued)

| SEQ ID NO | Peptide (14-mer) | OD | Seq. |
|---|---|---|---|
| 581 | L3-181 | 0.624 | GPC**RPRR**HSI**R**LLW |
| 582 | L3-182 | 0.818 | FYL**RERR**HQL**K**TEF |
| 583 | L3-183 | 0.768 | NMA**RSRK**NEC**K**YIA |
| 584 | L3-184 | 0.766 | DMD**KV KK**WVW**R**SFP |
| 585 | L3-185 | 0.794 | VLP**KERR**WLT**R**FLI |
| 586 | L3-186 | 0.799 | YEH**RHKR**FFH**K**DMP |
| 587 | L3-187 | 0.731 | HGH**KPRR**WIY**R**MPM |
| 588 | L3-188 | 0.738 | DGIR**DKK**CCW**R**DLI |
| 589 | L3-189 | 0.786 | ELV**KHRR**LDF**R**DPM |
| 590 | L3-190 | 0.792 | VHH**KLRR**VHL**R**LDV |
| 591 | L3-191 | 0.739 | GIN**RGRK**VSP**K**DEQ |
| 592 | L3-192 | 0.735 | NGY**RARK**ENL**K**FPE |
| 593 | L3-193 | 0.755 | AAY**RARK**IVE**K**SGW |
| 594 | L3-194 | 0.743 | WAP**RDRR**NMG**R**DPA |
| 595 | L3-195 | 0.7 | IED**KSKR**FNC**K**ECG |
| 596 | L3-196 | 0.746 | IEM**RYRR**DCS**R**FVN |
| 597 | L3-197 | 0.771 | QQI**KYKR**PTY**R**MVC |
| 598 | L3-198 | 0.78 | LPW**RERR**NGG**R**SSE |
| 599 | L3-199 | 0.781 | PQT**RYRK**YFY**R**PQM |
| 600 | L3-200 | 0.733 | HMC**RWRR**QIN**K**GVS |
| | **Spep-2 (con)** | **0.303** | **WTWKGRKSLLR** |

TABLE 13

| SEQ ID NO | Peptide | OD | Seq. |
|---|---|---|---|
| **601** | **L2-201** | **0.307** | RRR**RRRRRRR**R |
| **602** | **L2-202** | **0.314** | GGG**RKKR**RQR**R** |
| **603** | **L2-203** | **0.302** | GGR**KKRR**QRR**R** |
| **604** | **L3-201** | **0.315** | GGG**RKKR**RQR**R**RGG |
| **605** | **L3-202** | **0.301** | GGR**KKRR**QRR**R**GGG |
| **605** | **L2-204** | **0.311** | GGG**RRRRR**RRR**R** |
| | **Spep-2 (con)** | **0.303** | **WTWKGRKSLLR** |

<Example 14: Investigating acetylcholine receptor binding affinity of optimized peptides>

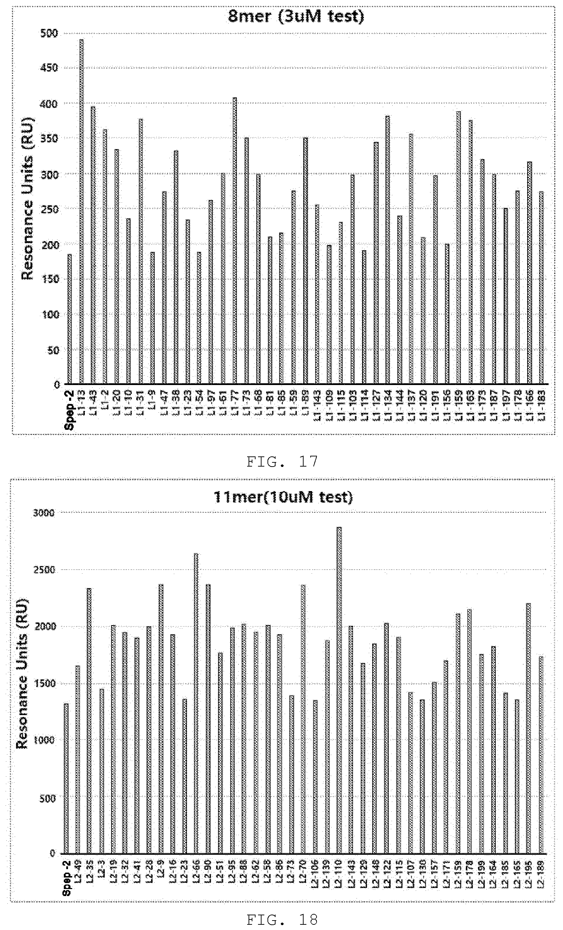

As for each library of the optimized peptides having excellent binding specificity to AchR in Example 13-3, forty peptides were selected and synthesize, and compared for AchR binding affinity (resonance unit: Ru) by using the surface plasmon resonance (SPR) analysis method in Example 2. The optimized peptides were tested at concentration conditions of 3 $\mu$M and 10 $\mu$M, and the results are shown in FIGS. 17 to 19. Spep-2 was used as a positive control. All of forty 8-mer (L1), 11-mer (L2), and 14-mer (L3) optimized peptides showed high binding affinity to AchR compared with the positive control Spep-2, and 20 peptides with high binding affinity to AchR were deduced for each library.

<Example 15: Investigating acetylcholine receptor inhibition of respective optimized peptides (L1, L2, L3) >

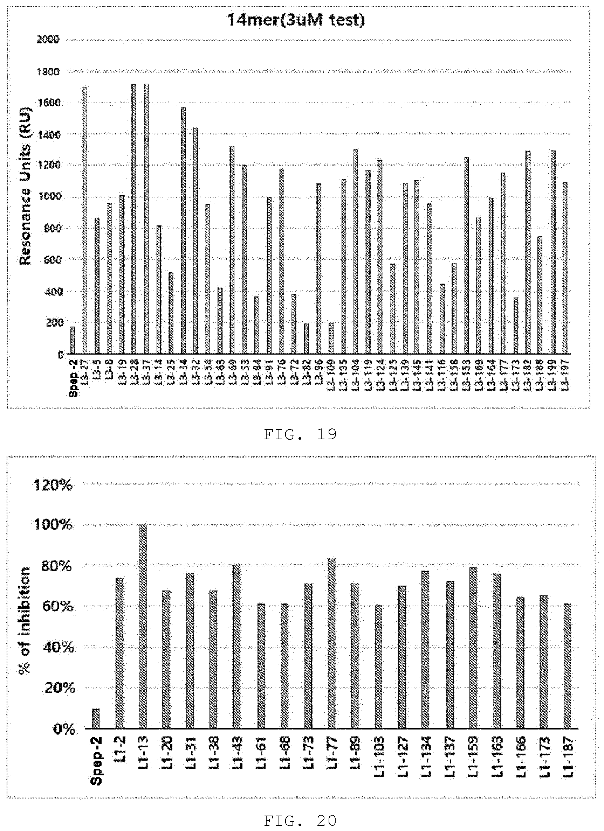

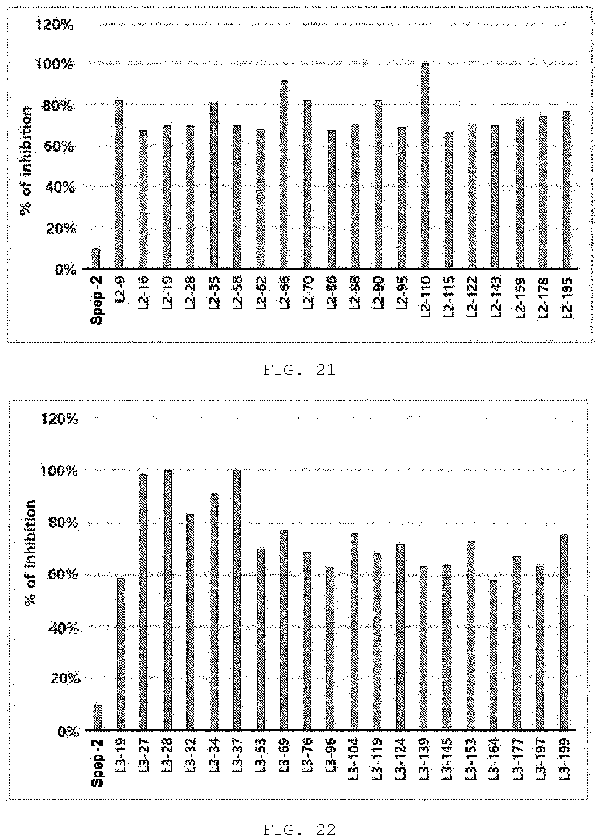

To investigate the AchR inhibitory effect of the 8-mer, 11-mer, and 14-mer peptides with excellent binding affinity to AchR identified in Example 14, the AchR inhibitory effect was investigated by the same method as in Example 3. The treatment with each of the peptides was conducted at 20 $\mu$M, and the treatment with Spep-2 as a control was also conducted. To investigate the excellent AchR inhibitory ability of the optimized peptides, a high concentration of nicotine was added at 400 $\mu$M or 600 $\mu$M, and the results are shown in FIGS. 20 to 22.

As shown in FIGS. 20 to 22, in cases of the addition of nicotine at a high concentration of 400 $\mu$M and the treatment with each peptide at 20 $\mu$M, the AchR inhibitory rate of Spep-2 as a control was about 10%, indicating little effect, whereas the AchR inhibitory rates of the 8-mer, 11-mer, and 14-mer peptides were all 50% or more, indicating excellent inhibitory effects compared with Spep-2. Out of these, the 8-mer L1-13, 11-mer L2-110, and 14-mer L3-27, 28, and 37 peptides showed an inhibitory effect close to 100%. Furthermore, it was verified that the AchR inhibitory rate of the peptides in cells were almost identical to the AchR binding affinity of each of the peptides in Example 14.

<Example 16: Investigating acetylcholine receptor binding affinity of representative peptides>

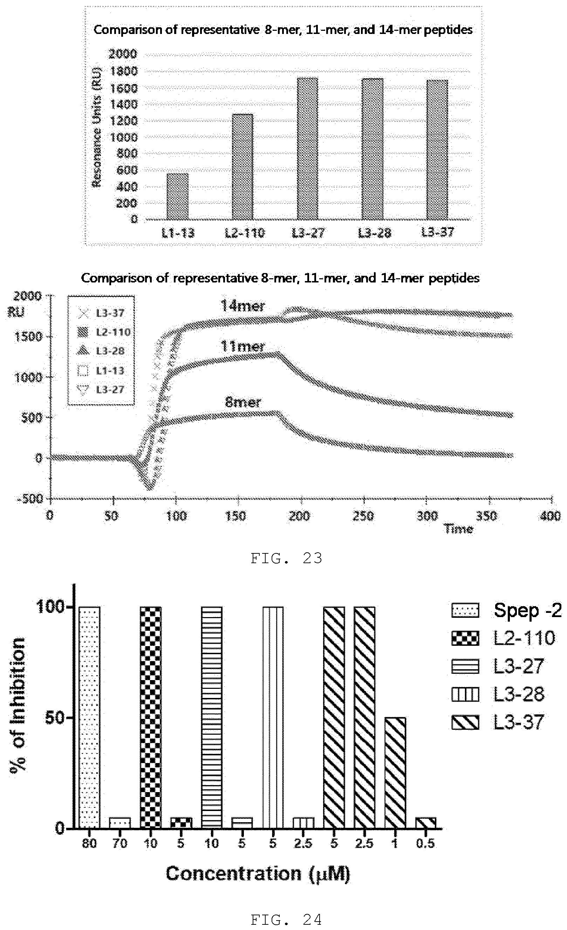

Representative peptides L1-13, L2-110, L3-27, L3-28, and L3-37 identified in Example 15 were compared for AchR binding affinity (resonance units: Ru) by using the surface plasmon resonance (SPR) analysis method in Example 2. The peptides were used at the same concentration condition of 3 $\mu$M. The results are shown in FIG. 23.

As shown in FIG. 23, the 11-mer L2-110 and the 14-mer L3-27, 28, and 37 showed higher binding affinity to AchR compared with the 8-mer L1-13, and the 14-mer L3-27, 28, and 37 showed the highest binding affinity. It was therefore verified that when the peptides of the formulas of the present invention were formed to be 8-mer or 11-mer, such peptides also showed high binding affinity to AchR, but the 14-mer peptides had the optimum binding affinity.

<Example 17: Investigating acetylcholine receptor inhibition of representative peptides L2-110, and L3-27, 28, and 37>

To investigate the AchR inhibitory effect of the L2-110, L3-27, L3-28, and L3-37 peptides identified to have excellent binding ability to AchR in Example 16, the AchR inhibitory effect was investigated by the same method as in Example 3. The treatment with each of the peptides was conducted at different concentrations, and the treatment with Spep-2 as a control was also conducted. The results are shown in FIG. 24.

As shown in FIG. 24, as for nicotine at a high concentration of 400 $\mu$M, Spep-2 inhibited AchR by 100% at 80 $\mu$M, but could not inhibit at a concentration of 70 $\mu$M. The 11-mer L2-110 and the 14-mer L3-27 inhibited AchR by 100% at 10 $\mu$M, but could not inhibit at 5 $\mu$M. The 14-mer L3-28 inhibited AchR by 100% at 5 $\mu$M, but could not inhibit at 2.5 $\mu$M. In addition, the 14-mer L3-27 inhibited AchR by 100% at 2.5 $\mu$M, and inhibited by 50% even at 1 $\mu$M.

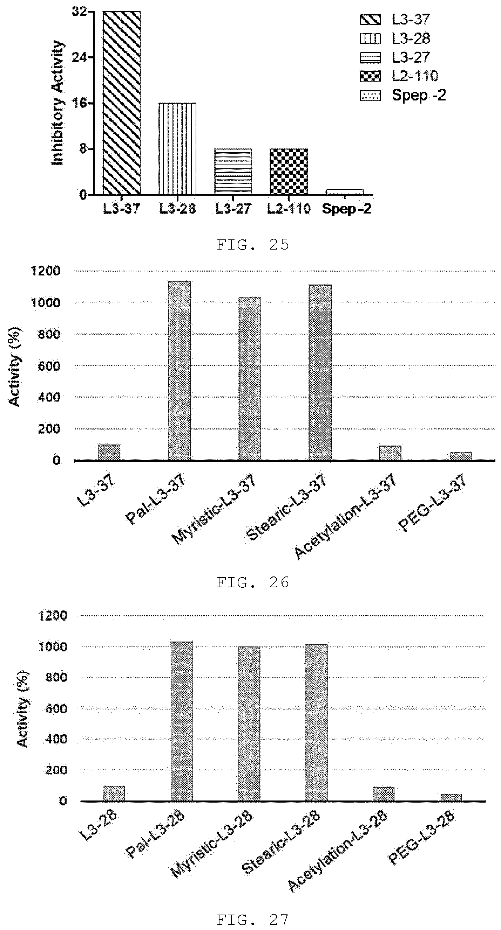

From the above results, the inhibitory ability on nicotinic acetylcholine receptors was expressed in multiples in FIG. 25. Compared with Spep-2, L2-110 and L3-27 showed an inhibitory effect improved by 8 times, L3-28 by 16 times, and L3-37 by 32 times. It was finally verified that the inhibitory effects of these peptides were also excellent in proportion with the binding ability to acetylcholine receptors in Example 15.

<Example 18: Investigating binding affinity to acetylcholine receptors according to various modifications at each terminus of representative peptides>

Each terminus of the L2-110, L3-27, L3-28, and L3-37 peptides identified in Example 17 was variously modified, and the change in AchR binding affinity was investigated therefor. Specifically, the peptides modified by the attachment of myristic acid or stearic acid in addition to palmitoyl, which are fatty acid derivatives, by the same method as in Example 8, or by acetylation or PEGylation, were compared with the basic peptide without modification. The results are shown in FIGS. 26 to 29.

As shown in FIGS. 26 to 29, when the fatty acid derivatives were attached to the terminus of the peptides according to the present invention, the binding affinity of all the peptides was increased by about 10 times (palmitoyl-, myristyl-, stearic-). The peptides modified by the attachment of palmitoyl showed higher binding affinity than the peptides with the attachment of other fatty acid derivatives.

The AchR binding affinity when the terminus of the peptides of the present invention was acetylated was similar to that when the terminus of the peptides was not modified. The AchR binding affinity when the terminus of the peptides of the present invention was PEGylated was reduced by about 50% compared with that when the termini of the peptides were not modified.

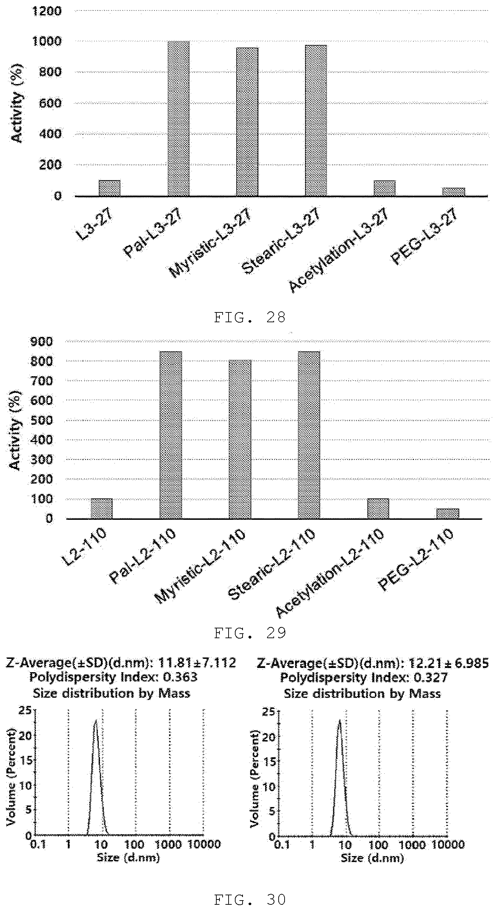

As shown in FIG. 30, the attachment of the fatty acid derivative meristic acid or stearic acid to the terminus of the peptides of the present invention induced the formation of a micelle structure, like the attachment of palmitoyl in Example 8.

<Example 19: Investigating acetylcholine receptor inhibition of Palmitoyl-L2-110, Palmitoyl-L3-27, Palmitoyl-L3-28, and Palmitoyl-L3-37>

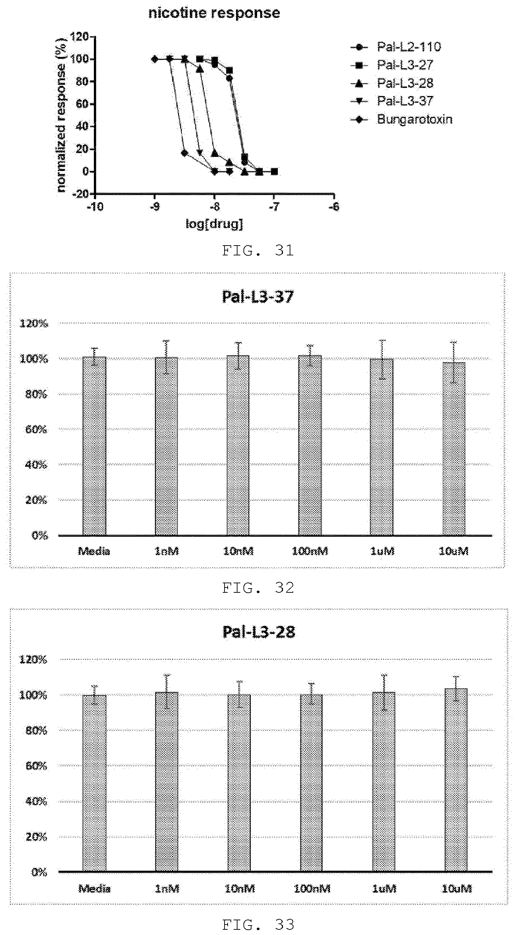

The AchR inhibitory effects of the Palmitoyl-L2-110, Palmitoyl-L3-27, Palmitoyl-L3-28, and Palmitoyl-L3-37 peptides with excellent binding affinity to AchR in Example 18 were investigated by the same method as in Example 3. Bungarotoxin was also used as a control. The results are shown in FIG. 31.

As shown in FIG. 31, the binding affinity to AchR was highest in Palmitoyl-L3-37, followed by Palmitoyl-L3-28, and Palmitoyl-L3-27 and Palmitoyl-L2-110 showed similar levels.

<Example 20: Investigating cytotoxicity of Palmitoyl-L2-110, Palmitoyl-L3-27, Palmitoyl-L3-28, and Palmitoyl-L3-37 peptides>

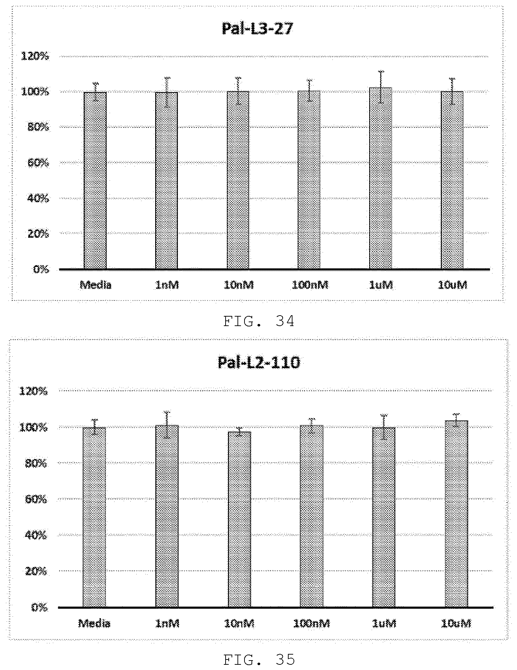

To evaluate cytotoxicity of the Palmitoyl-L2-110, Palmitoyl-L3-27, Palmitoyl-L3-28, and Palmitoyl-L3-37 peptides, WST assay was performed on TE671 cells, and the results are shown in FIGS. 32 to 35.

As shown in FIGS. 32 to 35, all the peptides showed no cytotoxicity even at a treatment concentration of 10 $\mu$M.

<Example 21: Analyzing in vivo efficacy of Palmitoyl-L3-37>

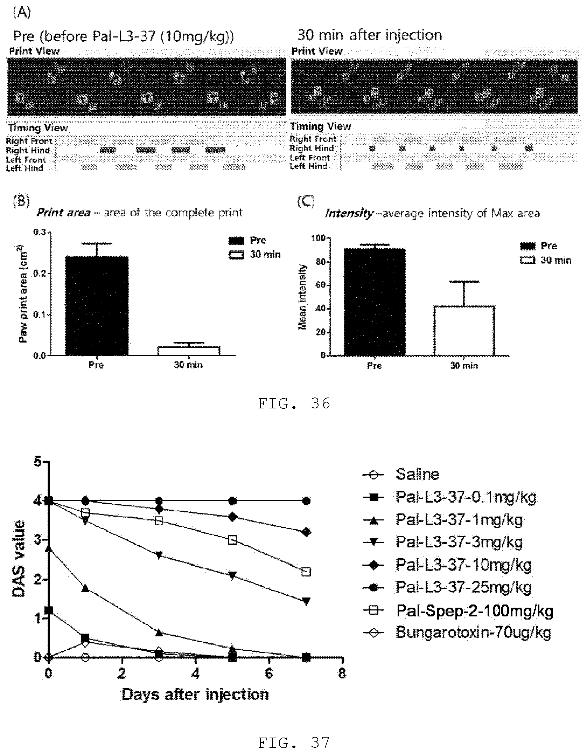

Animal efficacy assay was performed using Palmitoyl-L3-37, which had the highest binding affinity to acetylcholine receptors in Example 18. Female BALB/c mice aged 6 weeks were injected with Palmitoyl-L3-37 at a concentration of 10 mg/kg into the right hind thigh muscle (IM). The CatWalk data of the mice before injection and the CatWalk data of the mice 30 minutes after injection were compared and analyzed, and the results are shown in FIG. 36.

As shown in FIG. 36, the area where the sole of the right hind leg touched the floor was reduced after the injection of Palmitoyl-L3-37 compared with before the injection (A), and the area where the sole of the mouse touched the floor (B) and the average value of the maximum values of area (C) were also reduced after the injection of Palmitoyl-L3-37.

It can be therefore seen that Palmitoyl-L3-37 binds to acetylcholine receptors to thereby affect muscle relaxation in animals through the inhibition of acetylcholine.

<Example 22: Evaluating animal efficacy (ED50) and animal toxicity (LD50) of Palmitoyl-L3-37>

Animal efficacy (ED50) assay was conducted by digit abduction score (DAS) assay. The DAS assay was developed to measure the local muscle-weakening efficacy of a drug intramuscular (IM) injected to the mouse hind leg skeletal muscle, and Botox was also evaluated by the same method (Aoki, 1999).

Specifically, the DAS values for Palmitoyl-L3-37 in Example 18 were evaluated. Forty-five 6-week-old female BALB/c mice were randomly divided into: 5 groups, which were treated with Palmitoyl-L3-37 at 0.1 mg/kg, 1 mg/kg, 3 mg/kg, 10 mg/kg, 25 mg/kg; and a group treated with a saline solution (sterilized 0.9% NaCl) or 70 $\mu$g/kg bungarotoxin as a control group. Then, palmitoyl-L3-37 and the saline solution or bungarotoxin at the concentrations as above were injected each 50 ul into the hind thigh muscles of mice. Thereafter, the mice weight and the sole of the injected portion were observed and recorded every day, and the results are shown in FIGS. 37 to 39.

Referring to FIG. 37, as a result of observation for 7 days after injection, on 10 minutes (Day 0) after the administration of each sample, the Palmitoyl-L3-37 1mg/kg administration group was evaluated as DAS 2; and the Palmitoyl-L3-37 3mg/kg, 10mg/kg, and 25mg/kg administration groups were evaluated as DAS 4; and the Palmitoyl-L3-37 1mg/kg administration group and the saline solution or bungarotoxin administration group as a control were evaluated as DAS 0. It can be therefore seen that the efficacy (ED50) of Palmitoyl-L3-37 was 1 mg/kg in the animal test. In addition, during the 7-day observation, the DAS value was decreased and recovered in the other groups excluding the Palmitoyl-L3-37 25mg/kg group, but DAS4 was maintained in the Palmitoyl-L3-37 25mg/kg group.

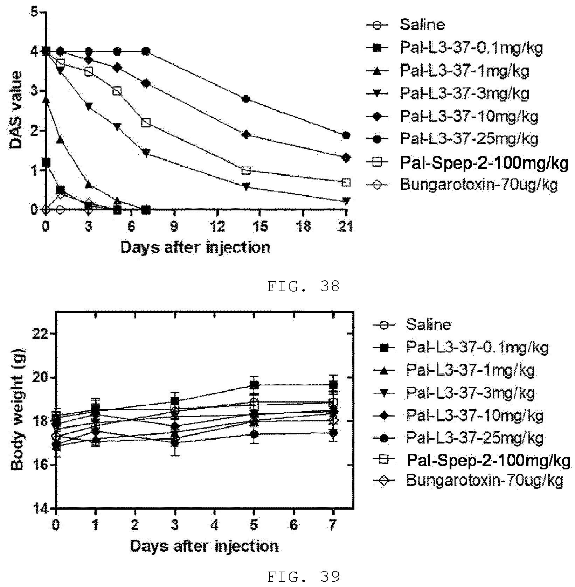

Referring to FIG. 38, as a result of observing the Palmitoyl-L3-37 25mg/kg administration group at weekly intervals until the 21st day after administration, the DAS value was gradually decreased after 7 days in the Palmitoyl-L3-37 25mg/kg administration group, and gradually decreased to 1 or less and recovered after 19 days of administration of Palmitoyl-L3-37. It can be therefore seen that the efficacy of Palmitoyl-L3-37 was reversible.

Last, as a result of evaluating the acute toxicity of Palmitoyl-L3-37 by measuring the weight every day for 7 days after administration (see FIG. 39), the weight of mice gradually increased for 7 days in all groups, and no toxicity was observed in the Palmitoyl-L3-37 administration group, and even at 25 mg/kg, which was the highest treatment concentration.

Moreover, the application relates to the following embodiments (items):

1. An acetylcholine receptor-binding peptide comprising an amino acid sequence set forth in Formula 1 below:

[Formula 1]

$X_L$-(K or R)-X-(K or R)-$X_M$-(K or R)-$X_N$

($X_L$, $X_M$, and $X_N$ each independently represent a sequence composed of one to eight arbitrary amino acids and X represents a sequence composed of one arbitrary amino acid).

2. The acetylcholine receptor-binding peptide of item 1, wherein the peptide comprises the amino acid sequence set forth in Formula 1 above, in which $X_L$, $X_M$, and $X_N$ each are independently composed of one to four arbitrary amino acids and X is composed of one arbitrary amino acid.

3. An acetylcholine receptor-binding peptide comprising an amino acid sequence set forth in Formula 2 below:

[Formula 2]

(K or R)-X-(K or R)-(K or R)-$X_{M-1}$-(K or R)

($X_{M-1}$ represents a sequence composed of one to three arbitrary amino acids and X represents a sequence composed of one arbitrary amino acid).

4. The acetylcholine receptor-binding peptide of item 3, wherein the peptide comprises the amino acid sequence set forth in Formula 2 above, in which $X_{M-1}$ is composed of three arbitrary amino acids and X is composed of one arbitrary amino acid.

5. The acetylcholine receptor-binding peptide of item 4, wherein the peptide is a peptide of any one sequence selected from the group consisting of SEQ ID NOs: 1 to 200.

6. An acetylcholine receptor-binding peptide comprising an amino acid sequence set forth in Formula 3 below:

[Formula 3]

$X_L$-(K or R)-X-(K or R)-(K or R)-$X_{M-1}$-(K or R)

($X_L$ and $X_{M-1}$ each independently represent a sequence composed of one to three arbitrary amino acids and X represents a sequence composed of one arbitrary amino acid) .

7. The acetylcholine receptor-binding peptide of item 6, wherein the peptide comprises the amino acid sequence set forth in Formula 3 above, in which $X_L$ is composed of one to three arbitrary amino acids, $X_{M-1}$ is composed of three arbitrary amino acids, and X is composed of one arbitrary amino acid.

8. The acetylcholine receptor-binding peptide of item 6, wherein the peptide comprises the amino acid sequence set forth in Formula 3 above, in which and $X_L$ and $X_{M-1}$ each are independently composed of three arbitrary amino acids and X is composed of one arbitrary amino acid.

9. The acetylcholine receptor-binding peptide of item 8, wherein the peptide is a peptide of any one sequence selected from the group consisting of SEQ ID NOS: 201 to 400.

10. An acetylcholine receptor-binding peptide comprising an amino acid sequence set forth in Formula 4 below:

[Formula 4]

$X_L$-(K or R)-X-(K or R)-(K or R)-$X_{M-1}$-(K or R)-$X_N$

($X_L$, $X_{m-1}$, and $X_N$ each independently represent a sequence composed of one to three arbitrary amino acids and X represents a sequence composed of one arbitrary amino acid).

11. The acetylcholine receptor-binding peptide of item 10, wherein the peptide comprises the amino acid sequence set forth in Formula 3, in which $X_L$ and $X_N$ each are independently composed of one to three arbitrary amino acids,

$X_{M-1}$ is composed of three arbitrary amino acids, and X is composed of one arbitrary amino acid.

12. The acetylcholine receptor-binding peptide of item 11, wherein the peptide comprises an amino acid sequence set forth in Formula 4 above, in which $X_L$, $X_{M-1}$, and $X_N$ each are independently composed of three arbitrary amino acids and X is composed of one arbitrary amino acid.

13. The acetylcholine receptor-binding peptide of item 12, wherein the peptide is a peptide of any one sequence selected from the group consisting of SEQ ID NOS: 401 to 600.

14. The acetylcholine receptor-binding peptide of any one of items 1 to 13, wherein the N-terminus or C-terminus of the peptide is modified.

15. The acetylcholine receptor-binding peptide of item 14, wherein the N-terminus or C-terminus is modified by palmitoylation, acetylation, amidation, formylation or PEGylation, or by a linkage of at least one selected from the group consisting of 2-mercaptoacetic acid, 3-mercaptopropionic acid, 6-mercaptohexanoic acid, pyroglutamic acid, succinimide acid, cystramine, cysteamine, methyl ester, ethyl ester, benzyl ester, myristic acid, stearic acid, palmitic acid, cholesterol, 6-amino hexanoic acid, and 8-amino octanoic acid.

16. A polynucleotide encoding the peptide of any one of items 1 to 13.

17. A cosmetic composition for wrinkle relief, the cosmetic composition comprising the peptide of any one of items 1 to 13.

18. A composition for preventing or treating an acetylcholine receptor hyperactivity-associated disease, the composition comprising the peptide of any one of items 1 to 13.

19. The composition of item 18, wherein the acetylcholine receptor hyperactivity-associated disease is at least any one selected from the group consisting of cervical dystonia, limb dystonia, truncal dystonia, blepharospasm, spasticity, hemifacial spasm, strabismus, nystagmus, tics, chronic pain, chronic migraine, neurogenic bladder, detrusor-sphincter dyssynergia, achalasia cardia, hyperhidrosis, and sialorrhea.

20. A health functional food composition for alleviating an acetylcholine receptor hyperactivity-associated disease, the composition containing the peptide of any one of items 1 to 13.

21. A composition comprising an acetylcholine receptor-binding peptide for a medical device, wherein the composition comprises the peptide of any one of items 1 to 13.

**Claims**

1. An acetylcholine receptor-binding peptide comprising an amino acid sequence set forth in Formula 4 below:

[Formula 4]

$$X_L-(K \text{ or } R)-X-(K \text{ or } R)-(K \text{ or } R)-X_{M-1}-(K \text{ or } R)-X_N$$

($X_L$, $X_{M-1}$, and $X_N$ each are independently composed of three arbitrary amino acids and X is composed of one arbitrary amino acid.)

2. The acetylcholine receptor-binding peptide of claim 1, wherein the peptide is a peptide of any one sequence selected from the group consisting of SEQ ID NOs: 401 to 600.

3. The acetylcholine receptor-binding peptide of claims 1 or 2, wherein the N-terminus or C-terminus of the peptide is modified.

4. The acetylcholine receptor-binding peptide of claim 3, wherein the N-terminus or C-terminus is modified by palmitoylation, acetylation, amidation, formylation or PEGylation, or by a linkage of at least one selected from the group consisting of 2-mercaptoacetic acid, 3-mercaptopropionic acid, 6-mercaptohexanoic acid, pyroglutamic acid, succinimide acid, cystramine, cysteamine, methyl ester, ethyl ester, benzyl ester, myristic acid, stearic acid, palmitic acid, cholesterol, 6-amino hexanoic acid, and 8-amino octanoic acid.

5. A polynucleotide encoding the peptide of any one of claims 1 to 2.

6. A cosmetic composition for wrinkle relief, the cosmetic composition comprising the peptide of any one of claims 1 to 2.

7. A composition for preventing or treating an acetylcholine receptor hyperactivity-associated disease, the composition comprising the peptide of any one of claims 1 to 2.

8. The composition of claim 7, wherein the acetylcholine receptor hyperactivity-associated disease is at least any one selected from the group consisting of cervical dystonia, limb dystonia, truncal dystonia, blepharospasm, spasticity, hemifacial spasm, strabismus, nystagmus, tics, chronic pain, chronic migraine, neurogenic bladder, detrusor-sphincter dyssynergia, achalasia cardia, hyperhidrosis, and sialorrhea.

9. A health functional food composition for alleviating an acetylcholine receptor hyperactivity-associated disease, the composition containing the peptide of any one of claims 1 to 2.

10. A composition comprising an acetylcholine receptor-binding peptide for a medical device, wherein the composition comprises the peptide of any one of claims 1 to 2.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

FIG. 26

FIG. 27

FIG. 28

FIG. 29

Z-Average(±SD)(d.nm): 11.81±7.112
Polydispersity Index: 0.363
Size distribution by Mass

Z-Average(±SD)(d.nm): 12.21±6.985
Polydispersity Index: 0.327
Size distribution by Mass

FIG. 30

## nicotine response

FIG. 31

FIG. 32

FIG. 33

FIG. 34

FIG. 35

(A)

Pre (before Pal-L3-37 (10mg/kg))    30 min after injection

(B) **Print area** – area of the complete print

(C) **Intensity** –average intensity of Max area

FIG. 36

Saline
Pal-L3-37-0.1mg/kg
Pal-L3-37-1mg/kg
Pal-L3-37-3mg/kg
Pal-L3-37-10mg/kg
Pal-L3-37-25mg/kg
Pal-Spep-2-100mg/kg
Bungarotoxin-70ug/kg

FIG. 37

FIG. 38

FIG. 39

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1216008
- KR 20180028748
- KR 20140139010

- KR 101971092
- KR 1020140139010